(19) **Europäisches Patentamt / European Patent Office / Office européen des brevets**

(11) **EP 4 109 463 A1**

(12) # EUROPEAN PATENT APPLICATION

(43) Date of publication:
**28.12.2022 Bulletin 2022/52**

(21) Application number: **21181585.7**

(22) Date of filing: **24.06.2021**

(51) International Patent Classification (IPC):
**G16H 30/40** (2018.01)   **G16H 50/20** (2018.01)
**G16H 50/70** (2018.01)

(52) Cooperative Patent Classification (CPC):
**G16H 50/20; G16H 30/40; G16H 50/70;**
G06N 20/00

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Siemens Healthcare GmbH
91052 Erlangen (DE)**

(72) Inventors:
• **HOELZER, Philipp
08536 Plainsboro (US)**
• **LIU, Siqi
08540 Princeton (US)**

Remarks:
Amended claims in accordance with Rule 137(2) EPC.

(54) **PROVIDING A SECOND RESULT DATASET**

(57)     The invention relates to a computer-implemented method, a providing system and a computer program product for providing a second result dataset, comprising or configured to receiving and/or determining a first result dataset, wherein the first result dataset is the output of an image-processing system processing a first medical image of a patient; receiving a modified first result dataset, wherein the modified first result dataset is based on a user modification of the first result dataset; receiving a second medical image of the patient, wherein the first medical image and the second medical image are of the same type; determining a second result dataset based on a comparison of the first result dataset and the modified first result dataset, and based on processing the second medical image with the image-processing system; providing the second result dataset.

FIG 3

**EP 4 109 463 A1**

**Description**

In the field of medical imaging an automatic detection and quantification of medical findings within the medical images (e.g., lung nodules within computed tomography or X-ray images) using image processing systems are state of the art for reducing reading time and increasing reading sensitivity. In particular, image processing systems based on machine learning or deep learning techniques are commonly used. For lung nodules such algorithms are of great clinical significance because nodules could potentially be cancerous and early intervention would considerably affect the patient outcome.

In many situations, the user of such automated systems is able to make manual corrections of detection errors such as false positive or false negatives by adding or deleting the system medical findings suggested by the image processing system. Additionally, classifications and/or quantifications (e.g., automatically generated nodule contours, marked regions of interest, or classification values) can also be manually changed to derive different classification and/or quantification results.

An important aspect of medical diagnosis are longitudinal studies to assess changes and disease progression between two different points in time, e.g., by comparing medical images acquired at those different points in time. Here, the automated systems can be used by processing classification and quantification tasks on the first and second images individually, and subsequently registering both images for nodule matching and trend analysis

However, when the user performs corrections of the results of the image processing system on the prior image (medical image at the earlier point in time), the same corrections by the user would often be needed in the output generated by the same system on a follow-up image (medical image at the later point in time). This is due to the fact that if imperfect algorithm performance on the first scan requires manual editing (adding nodules, rejecting nodules, modifying contours), there is a certain likelihood that the same tasks have to be performed on the second scan as well (given the similarity of images that were taken of the same patient). Such duplicated correction efforts could both increase the reading time as well as lowering the trust in the system.

So, it is an objective of the current invention to improve the automatic processing of medical images in a follow-up situation.

The problem is solved according to the independent claims. Further advantageous embodiments and additional advantageous features are described in the dependent claims and in the specification.

In the following, the solution according to the invention is described with respect to the claimed systems as well as with respect to the claimed methods. Features, advantages or alternative embodiments herein can be assigned to or implemented within the other corresponding claimed objects and vice versa. In other words, the systems can be improved with features described or claimed in the context of the corresponding method. In this case, the functional features of the methods are executed by the units of the systems.

In the following, the term "in particular" is used to indicate an optional and/or advantageous additional feature.

In a first aspect, the invention relates to a computer-implemented method for providing a second result dataset. The method is based on receiving and/or determining a first result dataset, wherein the first result dataset is the output of an image-processing system processing a first medical image of a patient.

In particular, the step of receiving the first result dataset can be executed by an interface, in particular, by an interface of a providing system, and the step of determining the first result dataset can be executed by a computation unit, in particular, by a computation unit of the providing system.

In particular, a medical images is an X-ray image, a computed tomography image (acronym "CT image"), a magnetic resonance image (acronym "MR image"), a positron emission tomography image (acronym "PET image"), a single-photon emission computed tomography (acronym "SPECT image"), and/or an ultrasound image (acronym "US image"). Furthermore, a medical image can be a microscopy image, histopathology image and/or a time-continuous biosignal analysis image.

In particular, a medical image can be a two-dimensional image, a three-dimensional image or a four-dimensional image. In particular, within a four-dimensional image, there are three spatial and one time dimensions.

A medical image can comprise a plurality of pixels or voxels, wherein the term "pixel" is mainly used for the building blocks of two-dimensional images, and the term is "voxel" is used for the building blocks of images with arbitrary dimension (mainly for three and more dimensions). However, in the following, the term "voxel" will be used as synonym for the term "pixel or voxel". Each voxel can comprise at least one intensity value corresponding to a certain tissue property (e.g., Hounsfield units for a CT image).

A medical image can be identical with or encapsulated in one or more DICOM files. Whenever DICOM is mentioned herein, it shall be understood that this refers to the "Digital Imaging and Communications in Medicine" (DICOM) standard, for example according to the current DICOM PS3.1 2020c standard (or any later or earlier version of said standard). It is also possible that several medical images are encapsulated in a single DICOM file.

In particular, a medical image can comprise additional metadata, e.g., a patient identifier, a patient name, an identifier of the modality used to acquire the medical image, a date and time of the image acquisition, an imaging protocol used during the acquisition of the medical image, and/or other metadate related to the medical image and/or its acquisition process.

An image processing system may comprise an algorithm working on input data an generating output data, wherein the input data is based at least partially on a medical image, and wherein the output data is indicative of medical findings within the medical image. In particular, the input data and output data can comprise additional data. In particular, the input data can comprise the medical image, and the output data can comprise medical findings related to the medical image. A synonym for the term "image processing system" is "computer-aided diagnosis system" (acronym "CAD").

An image processing system can be implemented directly within a medical imaging modality, on a dedicated server within the IT network of a hospital, or in the cloud. Alternatively, there can be a hybrid implementation combining such aspects (e.g., a image processing system can be implemented on the edge, meaning that the image processing system is executed within the IT network of a hospital and applied to data locally stored within the IT network of the hospital, but is managed and/or administrated from a cloud server).

In particular, the algorithm can be a machine learning algorithm based on a trained function. In particular, the machine learning algorithm can be trained based on training data comprising medical images and associated medical findings. Examples of machine learning algorithms are neural networks, in particular deep and/or convolutional neural networks, which can be trained based on a backpropagation algorithm.

A result dataset comprises a possibly empty set of characteristics associated with a certain medical image. A result dataset can be generated automatically or semi-automatically by the image processing system, but also manually based on user (e.g., a radiologist) input within a computer system.

In particular, the result dataset can comprise data characterizing the medical image or the patient being subject of the medical image. In particular, the result dataset can comprise measurements about structures within the medical image, or classifications of structures within the medical image. In particular, a result dataset can comprise medical findings.

A further step of the method according to the first aspect is receiving a modified first result dataset, wherein the modified first result dataset is based on a user modification of the first result dataset.

In particular, the step of receiving the modified first result dataset can be executed by an interface, in particular, by the interface of the providing system. The step of receiving the modified first result dataset can be executed before, after and/or in parallel to the step of receiving and/or determining the first result dataset.

A user modification of a result dataset is based on a user input related to the result dataset. In particular, a user modification can comprise adding a characteristic of the medical image to the result dataset, removing a characteristic of the medical image from the result dataset, changing a characteristic of the medical image within the result dataset, and/or confirming a characteristic of the medical image within the result dataset. The result of a user modification of a result dataset can be denoted as modified result dataset.

A further step of the method according to the first aspect is receiving a second medical image of the patient, wherein the first medical image and the second medical image are of the same type.

In particular, the step of receiving the second medical image can be executed by an interface, in particular, by the interface of the providing system. The step of receiving the second medical image can be executed before, after and/or in parallel to the step of receiving and/or determining the first result dataset. The step of receiving the second medical image can be executed before, after and/or in parallel to the step of receiving the modified result dataset.

A first medical image and a second medical image are of the same type, if they are acquired by the same type of imaging modality (e.g., a CT imaging modality) and relate to the same human or animal body region. It is not necessary that the first medical image and the second medical image were acquired with the same make or model of imaging modality or even the same imaging modality. Furthermore, it is not necessary that the first medical image and the second medical image have the same field of view.

For example, a first medical image being a computed tomography image of a human chest and a second medical image being a computed tomography image of a human chest are of the same type. However, if the second medical image would be an X-ray image of a human chest, the first medical image would not be of the same type as the second medical image, because they have been acquired with a different type of imaging modality. Furthermore, if the second medical image would be an computed tomography image of a human head, the first medical image would not be of the same type as the second medical image, because they relate to different body regions of a human.

A further step of the method according to the first aspect is determining a second result dataset based on a comparison of the first result dataset and the modified first result dataset, and based on processing the second medical image with the image-processing system.

In particular, the step of determining the second result dataset can be executed by a computation unit, in particular, by the computation unit of the providing system. In particular, the step of determining the second result dataset is executed after the previously described steps.

The last step of the method according to the first aspect is providing the second result dataset. In particular, the step of providing the second result dataset can be executed by an interface, in particular, by the interface of the providing system.

The inventors recognized that by using the proposed method user modifications related to a first result dataset can be automatically implemented within or transferred to a second result dataset. In a follow-up situation this implies that a user does not have to repeat certain user modifications that were already done with respect to the first medical image resulting in a faster diagnostic process, and/or that the quality of the diagnosis of the second medical image can be improved because errors of the image processing system with respect to the first medical image are already corrected within the second result dataset.

According to a further aspect of the invention, the method furthermore comprises receiving the first medical image and determining the first result dataset by processing the first medical image with the image-processing system. In particular, according to this aspect, the first result dataset is not received, but determined.

In particular, the step of receiving the first medical image is executed by an interface, in particular, by the interface of the providing system. In particular, the step of determining the first result dataset by processing the first medical image is executed by a computation unit, in particular, by the computation unit of a providing system.

The inventors recognized that, by processing both the first image and the second image with the image processing system, a higher consistency within the diagnostic process can be achieved, and the accuracy of the results can be increased.

According to a further aspect of the invention, the step of determining the second result dataset comprises generating the second result dataset by processing the second medical image with the image-processing system. In particular, the step of generating the second result dataset is executed with a computation unit, in particular, with the computation unit of the providing system.

According to this further aspect, the step of determining the second result dataset furthermore comprises modifying the second result dataset based on the comparison of the first result dataset and the modified first result dataset, wherein the step of modifying the second result dataset is executed after the step of generating the second result dataset. In particular, the step of modifying the second result dataset is executed with a computation unit, in particular, with the computation unit of the providing system.

The inventors recognized that, by modifying the second result dataset based on the comparison, the image processing system can remain unchanged. In particular, the image processing remains deterministic for its user, ensuring a reproducibility of the results of the image processing system.

According to a further aspect of the invention, the step of determining the second result dataset furthermore comprises determining a mapping between medical findings contained in the first result dataset and/or in the modified first result dataset and medical findings contained in the second result dataset. In other words, medical findings contained in the first result dataset and/or in the modified first result dataset are mapped to medical findings contained in the second result dataset and vice versa. In this aspect, the step of modifying the second result dataset is based on said mapping. In particular, the step of determining the mapping is executed with a computation unit, in particular, with the computation unit of the providing system.

According to this aspect, preferably the medical findings are candidate structures, and more preferably the candidate structures are indicative of candidate lesions (or, in other words, possible lesions) within the patient.

In general, a medical finding is a clinically significant observation, in particular, based on a physical examination, a medical imaging procedure or a laboratory test. In particular, a medical finding can be one of the characteristics contained in a result dataset. A medical finding can be a quantitative finding (also denoted as "measurement" or "classification"), e.g., "heart rate is 85 bpm", or a qualitative finding, e.g., "the patient has lung cancer". A medical finding related to the patient's medical signs and symptoms evolution can be denoted as clinical finding. A medical finding related to an intermediate biological biomarker can be denoted as physiological finding. A medical finding related to the physical damage produced by a disease can be denoted as pathological or histopathological finding. Medical finding can relate to a targeted medical test or to an unrelated exploration (incidental finding). In particular, a medical finding can be determined by the image processing system when being applied to a medical image. In other word, the image processing system produces a (potentially empty) set of medical findings when using a medical image as input data. Alternatively, a medical finding can also be determined by a user and entered via an user interface.

A candidate structure is a medical finding related to a potentially abnormal structure within the medical body. In particular, a candidate structure is depicted in a medical image. In particular, a candidate structure can be a candidate lesion. A medical finding being a candidate structure can comprise position or a location of the potentially abnormal structure within a medical image. A medical finding being a candidate structure can furthermore comprise analytical information about the potentially abnormal structure. The analytical information may include at least one of feature information, class information, diagnostic result information and morphometry information. The feature information may include one or more feature categories, and one or more features corresponding to each feature category. For example, the feature information may include feature categories of shape, margin, echo pattern, orientation and boundary of the candidate structure, and the feature category of "shape" may include features of an irregular shape, a round shape and an oval shape. The diagnostic result information may indicate a result of a determination as to whether a candidate structure is probably abnormal or not (e.g., whether a lesion is benign or malignant). The class information refers to a class level of a corresponding candidate structure (e.g., a degree of benignancy or malignancy of the lesion). The morphometry information refers to a mask or a segmentation of a corresponding candidate structure.

A mapping between medical findings in the first result dataset and in the second result dataset is a correspondence between one or less medical findings in the first result dataset and one or less medical findings in the second result dataset. In other words, every medical finding in the first result dataset is mapped to either one or none of the medical findings in the second result dataset, and every medical finding in the second result dataset is mapped to either one or none of the medical findings in the first result dataset.

In particular, if at least some of the medical findings are candidate structures and comprise a location of the respective structure in the respective medical image, a mapping can be based on a mapping of the respective locations contained in the respective candidate structures.

The inventors recognized that a mapping between medical findings is a very efficient way to compare the first result dataset and the second result dataset.

According to a further aspect of the invention the step of determining the second result dataset furthermore comprises determining a registration between the first medical image and the second medical image based on the first result dataset and the second result dataset, and/or based on the first medical image and the second medical image. According to this aspect the mapping between medical findings contained in the first result dataset and medical findings contained in the second result dataset is based on said registration. In particular, the step of determining the registration is executed with a computation unit, in particular, with the computation unit of the providing system.

In particular, a registration is a function which maps a first medical image to second medical image. In particular, the registration function assigns for a subset of pixels or voxels of the first medical image corresponding pixels or voxels in the second medical image, which correspond to the same physical location within a patient. In particular, the first medical image and the second medical image have the same dimensionality.

The registration can be an intensity-based registration and/or a feature-based registration. The registration can be based on a linear (or affine) transformation or based on a non-linear transformation. A non-linear transformation can be based on radial basis functions, physical continuum models and/or large deformation models (e.g., diffeomorphisms). A registration can be based on a frequency-domain representation of the first and/or the second medical image, e.g., a Fourier or a Laplace transformation of the first and/or the second medical image. A registration can be determined manually, interactively, semi-automatically or automatically. In particular, a registration can be determined by applying a trained registration function (e.g., a convolutional or a non-convolutional neural network) based on known training registrations of pairs of training images.

The inventors recognized that based on a registration a reliable mapping between medical findings in the first result dataset and the second result dataset can be achieved, even in situations where the pose and/or the internal structure of the patients varies in the acquisition of the first medical image and of the second medical image.

According to a further aspect of the invention the registration and/or the mapping is based on a deformable transformation. Preferably the registration and/or the mapping is based on a vector momentum-parameterized stationary velocity field.

In general, a non-deformable registration preserves value of the Euclidean distance of two points, so that d(TF(x), TF(y)) d(x, y), where d(x, y) is the distance of two points, and TF(x) is the result of applying the registration to a point. A deformable registration does not preserve the value of the Euclidean distance of two points.

In particular, the deformable registration can be a radial basis function (in particular, selected from the group of thin-plate transformations or surface spline transformations, multiquadric transformations, and compactly-supported transformations), physical continuum models (e.g., viscous fluid models), and large deformation models (diffeomorphism transformations) .

The inventors recognized that by using a deformable registration local geometric differences between the first medical image and the second medical image can be considered. In particular, such local geometric differences can occur due to physical changes in the patient in-between the first and the second medical imaging examination, or due to different poses of the patient when performing the first and the second medical imaging examination.

An acronym for vector momentum-parameterized stationary velocity field is "vSVF". Methods for vSVF are known e.g. from Z. Shen et al., "Networks for Joint Affine and Non-Parametric Image Registration" (20019) 4219-4228. 10.1109/CVPR.2019.00435. In particular, vSVF is a fluid dynamic method that deforms the image according to a smooth velocity field, where the deformation map can be accumulated along the time.

The inventors recognized that using vSVF techniques registrations can be determined in a faster way, with a better control of transformation regularity, than other comparable registration techniques.

According to a further aspect of the invention, the step of modifying the second result dataset comprises at least one of the steps of: inserting a first medical finding into the second result dataset, and/or removing a second medical finding from the second result dataset, and/or altering a third medical finding within the second result dataset. In particular, the step of inserting and/or the step of removing and/or the step of altering is executed with a computation unit, in particular, with the computation unit of the providing system.

In particular, by modifying the second result dataset a modified second result dataset is automatically created without a user modification.

The inventors recognized that by a modification of the second result dataset the user modifications related to the first result dataset can be transformed to the second result dataset. In particular, false positives and false negatives can be corrected automatically.

According to a further aspect of the invention, the step of modifying the second result dataset comprises determining a first region within the first medical image and a second region within the second medical image, wherein the first region and the second region correspond to the first medical finding to be inserted, and/or to the second medical finding to be removed, and/or to the third medical finding to be altered. According to this aspect, the step of modifying the second result dataset furthermore comprises determining a similarity score between the first region and the second region. Here, the step of inserting the first medical finding, and/or the step of removing the second medical finding, and/or the step of altering the third medical finding is executed only if the similarity score fulfills a predefined criterion.

In particular, the steps of determining the first region and the second region and of determining the similarity score are executed with a computation unit, in particular, with the computation unit of the providing system.

In particular, the region of a medical finding corresponds to an associated region within the respective medical image, or the corresponding region in the other medical image. In other words, the region of a medical finding in the first medical image corresponds to an associated region within the first medical image or to an corresponding region in the second medical image, and the region of a medical finding in the second medical image corresponds to an associated region within the second medical image or a corresponding region in the first medica image. In particular, the first region can be determined based on the second region by applying the registration and/or the transformation, and vice versa.

In particular, the first region and the second region can be a rectangular (for two-dimensional images) or a cuboid (for three-dimensional images) region comprising the medical finding or related to the medical finding. In particular, the first region and the second region can have the same size (measured in terms of numbers of pixels and or in terms of voxels).

In particular, the similarity score can be a single number, or a vector comprising several numbers. In particular, the similarity score can be probability value, wherein a low similarity score corresponds to a low probability that the first region and the second region are matching and/or have not been altered between the first time and the second time, and a high similarity score corresponds to a high probability that the first region and the second region are matching and/or have not been altered between the first time and the second time. In case the similarity score is a vector of numbers, each element of the vector can be a probability score (e.g., rating different aspects of similarity).

In particular, the predefined criterion can be based on a threshold, the threshold having the same structure as the similarity score. If both the similarity score and the threshold are numbers, the predefined criterion can be fulfilled either if the similarity score is larger than (or equal to) the threshold, or if the similarity score is smaller than (or equal to) the threshold. If both the similarity score and the threshold are vectors of numbers, the predefined criterion can be fulfilled if for a predefined number of entries of the vectors the respective entries of the similarity score vector are larger (or smaller) than the respective entries of the threshold vector.

The inventors recognized that by modifying the second result dataset only in cases where the similarity score between the first region and the second region fulfills a predetermined criterion, user modifications of the first result dataset are not automatically transferred to the second result dataset if the underlying medical images diverge in the underlying areas. Those divergencies can occur in the case of a time progression of the medical finding or if there is a new actual medical finding. For example, if a structural change occurs at the location of a false positive medical finding in the first medical image (e.g., a false positive candidate lesion), this should not be automatically marked as false positive in the follow-up, but again be reviewed by a user or physician.

According to a further possible aspect of the invention, the step of determining the similarity score comprises using the first region and the second region as input data for a similarity-detecting machine learning algorithm. In particular, the similarity-detecting machine learning algorithm is based on training data comprising pairs of original regions and transformed regions, the transformed regions being based on applying an image transformation to the original region. In particular, the training data furthermore comprises pairs of original regions and unrelated regions.

In general, a machine learning algorithm mimics cognitive functions that humans associate with other human minds. In particular, by training based on training data the machine learning algorithm is able to adapt to new circumstances and to detect and extrapolate patterns.

In general, parameters of a machine learning algorithm can be adapted by means of training. In particular, supervised training, semi-supervised training, unsupervised training, reinforcement learning and/or active learning can be used. Furthermore, representation learning (an alternative term is "feature learning") can be used. In particular, the parameters of the machine learning algorithm can be adapted iteratively by several steps of training.

In particular, a machine learning algorithm can comprise a neural network, a support vector machine, a decision tree and/or a Bayesian network, and/or the trained function can be based on k-means clustering, Q-learning, genetic algorithms and/or association rules. In particular, a neural network can be a deep neural network, a convolutional neural network or a convolutional deep neural network. Furthermore, a neural network can be an adversarial network, a deep adversarial network and/or a generative adversarial network.

The similarity-detecting machine learning model is trained based on pairs of original regions and transformed regions. In particular, an original region can be extracted from a medical image, and a transformed region can be extracted from a transformation of the same medical image at a position corresponding to the original region, wherein the transformation mimics changes between a first and a second medical image that are not due to pathological changes (e.g., different patient positioning or different imaging geometries). The similarity-detecting machine learning model can also be trained based on pairs of original regions and unrelated regions. In particular, an unrelated region can be extracted from a different medical image of the same patient or a different patient than the original region. The unrelated region can also correspond to a pathological change of a medical finding in the original lesion and be extracted from the associated follow-up medical image.

In particular, the original region and the transformed region are of the same dimension and the same size (measured in number of pixels or voxels). In particular, the first region and the second region also have the same size (measured in number of pixels and voxels) as the original region and the transformed region. In particular, during the training of the similarity-detecting machine learning model, the original region and the transformed region are used as input for the similarity-detecting machine learning model, and parameters of the machine learning model are adapted based on the difference of the output of the similarity-detecting machine learning model and a target value (in particular, the target value 1 indicating a total similarity). In particular, during the training of the machine learning model, a pair comprising an original region and an unrelated region are used as input for the similarity-detecting machine learning model, and the parameters of the machine learning model are adapted based on the difference of the output of the similarity-detecting machine learning model and a target value (in particular, the target value 0 indicating no similarity). Adapting parameters can be based on minimizing a cost function, in particular, using the backpropagation algorithm.

In determining the similarity score, the first region and the second region are used as input for the similarity-detecting machine learning model, and the similarity score is equal to or based on the output of the similarity-detecting machine learning model.

The inventors recognized that using a similarity-detecting machine learning model, all features corresponding to the first and the second region can be considered for creating the similarity score. In particular, also correlations not recognized by a human expert can be utilized for inferring the similarity score. By training based on original regions and transformed regions it can be achieved that the similarity-detecting machine learning model can also correctly recognize non-pathological changes between the first region and the second region and assign a high similarity.

According to a further aspect of the invention, the modified first result dataset comprises a false-negative medical finding not contained in the first result dataset, and the first medical finding corresponds to the false-negative medical finding. Alternatively, or additionally, the first result dataset comprises a false-positive medical finding not contained in the modified first result dataset, and the second medical finding corresponds to the false-positive medical finding. Alternatively, or additionally, the modified first result dataset comprises a modified medical finding corresponding to a modification of an original medical finding contained in the first result dataset, and the third medical finding corresponds to the original medical finding, and the step of altering the third medical finding is performed in accordance with the modification of the original medical finding.

A false negative medical finding corresponds to a result of the image processing system which wrongly indicates that a certain medical finding is not present, wherein in fact the medical finding is present. As a consequence, there is no corresponding medical finding contained in the first result dataset, and the false negative medical finding is only contained in the modified first result dataset (due to a user modification of including the medical finding). For example, a false negative medical finding can correspond to a lesion not being detected by the image processing system. Another term for false negative medical finding is "type II error".

A false positive medical finding corresponds to a result of the image processing system which wrongly indicates that a certain medical finding is present, wherein in fact the medical finding is not present. As a consequence, there is no corresponding medical finding contained in the modified first result dataset, and the false positive medical finding is only contained in the first result dataset (due to a user modification of removing the medical finding), For example, a false positive medical finding can correspond to a lesion being detected by the image processing system in error. Another term for false positive medical finding is "type I error".

A original medical finding corresponds to a result of the image processing system which correctly indicates the presence of a certain medical finding, but indicates a wrong further property of the certain medical finding. As a consequence, the original medical finding is contained in the first result dataset, and the modified first result dataset comprises the modified medical finding as a replacement of the original medical finding (due to a user modification changing the further property of the original medical finding). For example, a modified medical finding can correspond to an altered classification of a lesion being detected but wrongly classified by the image processing system.

The inventors recognized that false positive medical findings, false negative medical findings and modifications from an original medical finding to a modified medical finding can be detected reliably by an automated process, so that there can be a reliable second result dataset.

According to a further possible aspect of the invention, the modified first result dataset comprises a false-negative medical finding not contained in the first result dataset. Furthermore, the step of modifying the second result dataset comprises inserting a first medical finding corresponding to the false negative medical finding into the second result dataset.

According to a further possible aspect of the invention, the first result dataset comprises a false-positive medical finding not contained in the modified first result dataset. Furthermore, the step of modifying the second result dataset comprises removing a second medical finding corresponding to the false-positive medical finding from the second result dataset.

According to a further possible aspect of the invention, the modified first result dataset comprises a modified medical finding corresponding to a modification of an original medical finding contained in the first result dataset. Furthermore, the step of modifying the second result dataset comprises altering a third medical finding corresponding to the original medical finding within the second result dataset in accordance with the modification of the original medical finding

According to a further aspect of the invention, the step of determining the second result dataset comprises modifying the image-processing system based on the comparison of the first result dataset and the modified first result dataset. Furthermore, the step of determining the second result dataset comprises generating the second result dataset by processing the second medical image with the image-processing system, wherein the step of generating the second result dataset is executed after the step of modifying the image-processing system. In particular, the steps of modifying the image-processing system and of generating the second result dataset are executed with a computation unit, in particular, with the computation unit of the providing system.

In particular, modifying the image processing system can comprise modifying a parameter of the image processing system. In particular, modifying the image processing system can be based on a training algorithm, for example, based on training data comprising the first result dataset and the modified first result dataset, or comprising a comparison result of the comparison of the first result dataset and the modified first result dataset. The modification of the image processing system can be a permanent modification or a temporary modification. In particular, the modification of the image processing system is a temporary modification only used for generating the second result dataset. In other words, the image processing system is temporary modified for each execution of the method according to the invention and its aspects.

The inventors recognized that, by modifying the image processing system based on said comparison, information from the user modifications can directly be integrated into the image processing system so that no comparison of result datasets is necessary. Furthermore, by a modification of the image processing system based on the comparison, real changes between the first medical image and the second medical image can be likely detected by the modified image processing system.

According to a further aspect of the invention, the image processing system comprises a trainable machine-learning algorithm, and the step of modifying the image processing system comprises at least one of overfitting the trainable machine-learning algorithm based on the comparison of the first result dataset and the modified first result dataset; and/or altering the operating point of the trainable machine-learning algorithm based on the comparison of the first result dataset and the modified first result dataset; and/or conditioning the output of the trainable machine-learning algorithm based on the comparison of the first result dataset and the modified first result dataset.

In particular, overfitting of the trainable machine-learning algorithm comprises performing training on a certain set of training data so that the trainable machine-learning data better fits said set of training data, but fits other data being not similar to said set of training data worse. In the present case, overfitting the trainable machine-learning algorithm can comprise using the first medical image and/or the second medical image as training data for the image processing systems in a plurality of steps, while using the modified first result dataset as ground truth (i.e., adapting parameters of the trainable machine-learning algorithm based to minimize the difference between the actual output and the ground truth). The inventors recognized that by overfitting the trainable machine-learning algorithm it can be trained to mimic the user modifications on a medical image being similar to the first medical image, so that those user modifications are implemented when applying the image processing system to the second medical image.

In particular, altering the operating point of the machine learning algorithm comprises changing a classification threshold or a decision threshold. In particular, for a given operating point (or a given classification / decision threshold), there is a false positive rate (the probability of a false positive detection) and a false negative rate (the probability of a false negative detection), which depend on the operating point. Altering the operating point increases the false positive rate and decreases the false negative rate, or vice versa. The inventors recognized that, by altering the operating point, the false positive rate and the false negative rate can be adapted to mimic the user modifications. For example, if the user modifications indicate the presence of several false positive medical findings, the operating point can be changed so that the machine-learning algorithm and the image processing system is less sensitive (e.g., increasing the false negative rate and decreasing the false positive rate).

According to a further possible aspect, the invention relates to a computer-implemented method for providing a second result dataset, comprising:

- receiving and/or determining a first result dataset, wherein the first result dataset is the output of an image-processing system processing a first medical image of a patient,
- receiving a modified first result dataset, wherein the modified first result dataset is based on a user modification of the first result dataset,
- receiving a second medical image of the patient, wherein the first medical image and the second medical image are of the same type,
- modifying the image-processing system based on the modified first result dataset, or based on a comparison of the first result dataset and the modified first result dataset,
- generating the second result dataset by processing the second medical image with the modified image-processing system,
- providing the second result dataset.

In particular, the step of modifying the image-processing system comprises at least one of:

- overfitting the trainable machine-learning algorithm based on the modified first result dataset, or based on a comparison of the first result dataset and the modified first result dataset,
- altering the operating point of the trainable machine-learning algorithm based on the modified first result dataset, or based on a comparison of the first result dataset and the modified first result dataset,; and/or
- conditioning the output of the trainable machine-learning algorithm based on the modified first result dataset, or based on a comparison of the first result dataset and the modified first result dataset.

According to a further possible aspect of the invention, the step of providing the second result dataset comprises providing an indication about modifications of the second result dataset and/or modifications of the image-processing system.

An indication about a modification can comprise displaying a message or an alert for the user by an interface. In particular, a certain medical finding of the second result dataset can be displayed in a highlighted form in case it was subject to an automatic modification. For example, said certain medical findings can be displayed with a border of a certain color, with a border of a certain structure (e.g., with thicker border or with a different line style than usual) or with a certain mark indicating that said certain medical finding was altered with respect to the output of the (unmodified) image-processing system.

The inventors recognized that such an indication can be used to alert the user that certain automatic modifications have been executed based on prior user modifications.

In a second aspect the invention relates to a providing system for providing a second result dataset comprising an interface and a computation unit,

- wherein the interface and/or the computation unit are configured for receiving and/or determining a first result dataset, wherein the first result dataset is the output of an image-processing system processing a first medical image of a patient;
- wherein the interface is configured for receiving a modified first result dataset, wherein the modified first result dataset is based on a user modification of the first result dataset;
- wherein the interface is configured for receiving a second medical image of the patient, wherein the first medical image and the second medical image are of the same type;
- the computation unit is configured for determining a second result dataset based on a comparison of the first result dataset and the modified first result dataset, and based on processing the second medical image with the image-processing system (IPS),
- wherein the interface is configured for providing the second result dataset.

In particular, the providing system can be configured to execute the method for providing a second result dataset according to the invention and its aspects. The providing system is configured to execute the method and its aspects by its interface and the computation unit being configured to execute the respective method steps.

In a third aspect, the invention relates to a computer program product or a computer-readable storage medium comprising instructions which, when the program is executed by a computer, cause the computer to carry out the method according to the invention and its aspects.

The realization of the invention or one of its aspects by a computer program product and/or a computer-readable medium has the advantage that already existing servers, devices and clients can be easily adapted by software updates in order to work as proposed by the invention.

The said computer program products can be, for example, a computer program or comprise another element apart from the computer program. This other element can be hardware, for example a memory device, on which the computer program is stored, a hardware key for using the computer program and the like, and/or software, for example a documentation or a software key for using the computer program.

The properties, features and advantages of this invention described above, as well as the manner they are achieved, become clearer and more understandable in the light of the following description and embodiments, which will be described in detail in the context of the drawings. This following description does not limit the invention on the contained embodiments. Same components or parts can be labeled with the same reference signs in different figures. In general, the figures are not for scale.

The numbering and/or order of method steps is intended to facilitate understanding and should not be construed, unless explicitly stated otherwise, or implicitly clear, to mean that the designated steps have to be performed according to the numbering of their reference signs and/or their order within the figures. In particular, several or even all of the method steps may be performed simultaneously, in an overlapping way or sequentially.

In the following:

Fig. 1     displays embodiments of medical images and medical findings,
Fig. 2     displays embodiments of medical images and other medical findings,
Fig. 3     displays a data flow diagram according to an embodiment of the invention,
Fig. 4     displays a data flow diagram according to another embodiment of the invention,
Fig. 5     displays a first embodiment of an infrastructure for methods and systems according to the invention,
Fig. 6     displays a second embodiment of an infrastructure for methods and systems according to the invention,
Fig. 7     displays a flowchart of a first embodiment of the method for providing a second result dataset,
Fig. 8     displays a flowchart of a second embodiment of the method for providing a second result dataset,
Fig. 9     displays a flowchart of a third embodiment of the method for providing a second result dataset,
Fig. 10    displays a flowchart of a fourth embodiment of the method for providing a second result dataset,
Fig. 11    displays a flowchart of a fifth embodiment of the method for providing a second result dataset,
Fig. 12    displays a flowchart of a sixth embodiment of the method for providing a second result dataset,
Fig. 13    displays a providing system for providing a second result dataset.

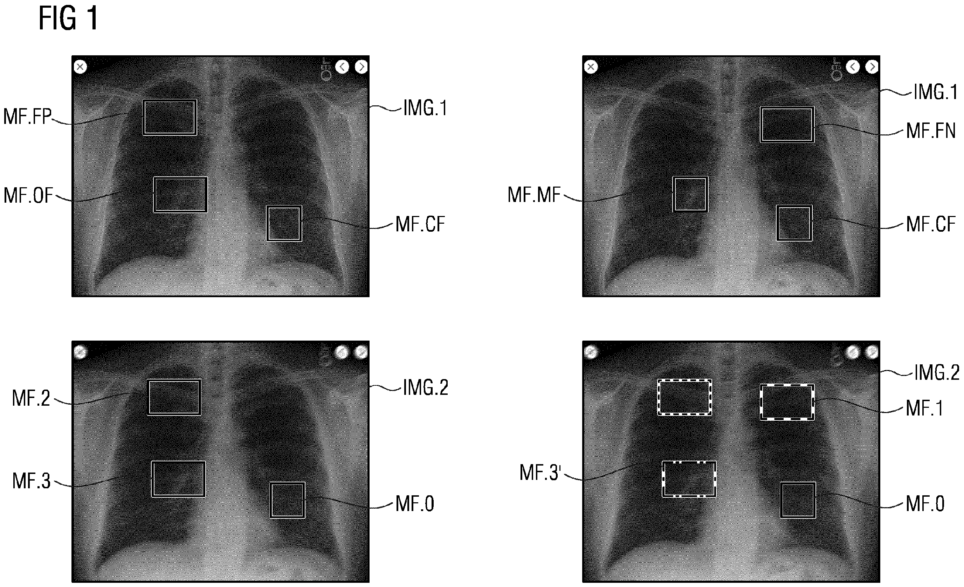

Fig. 1 displays embodiments of medical images IMG.1, IMG.2 and medical findings MF.FP, MF.FN, MF.OF, MF.MF, MF.CF, MF.0, ..., MF.3.. Here the medical images IMG.1, IMG.2 are two-dimensional X-ray images of a chest of a human, wherein the first medical image IMG.1 was acquired at a first time, and the second medical image IMG.2 was acquired at a second time. The medical findings MF.FP, MF.FN, MF.OF, MF.MF, MF.CF, MF.0, ..., MF.3 are candidate lesions areas marking a bounding box for a potential lung lesion.

Within the first medical image IMG.1, a set of medical findings MF.FP, MF.OF, MF.CF corresponding to candidate lesion areas were detected by an image processing system IPS. A user, for example a radiologist, can perform user modifications UM related to these medical findings. For example, the user can modify an original finding MF.OF to become a modified medical finding MF.MF, delete a false positive medical finding MF.FP, and/or include a false negative medical finding MF.FN.

The second medical image IMG.2 was acquired at a later time than the first medical image IMG.1, potentially at a different location and with a different modality, e.g., as part of a follow-up procedure. In particular, due to differences in the patient modification or in the imaging geometry of the imaging devices, it can be necessary to use a registration between the first medical image IMG.1 and the second medical image IMG.2 in order to find a transformation between coordinates within the first medical image IMG.1 and coordinates within the second medical image IMG.2.

If the (potentially modified) image processing system IPS is applied to the second medical image IMG.2, another set of medical findings MF.0, ..., MF.3 corresponding to candidate lesions is generated, which is similar to the original set of MF.FP, MF.OF, MF.CF within the first medical image IMG.1 if there are no significant anatomical changes within the patient between the first medical image IMG.1 and the second medical image. As a consequence, the image processing system IPS would in the second medical image IMG.2 miss a first medical finding MF.1 related to the false negative finding MF.FN, mistakenly find a second medical finding MF.2 related to the false positive finding MF.FP and/or incorrectly classify and/or quantify a third medical finding MF.3 related to the original medical finding MF.OF.

By methods and systems according to the invention and its embodiments, the medical findings MF.0, ...., MF.4 related to the second medical image IMG.2 can be adopted automatically based on the user modifications UM related to the medical findings MF.FP, MF.OF, MF.CF within the first medical image IMG.1, and a modified set of medical findings MF.0, ...., MF.4 can be provided to the user. In particular, the user can be notified about such adoptions. In the displayed embodiment, medical findings MF.1 related to a prior false negative finding MF.FN are displayed in a first way (here, using dashed lines), medical findings MF.2 related to a prior false positive finding MF.FP are displayed in a second way (here, using dotted lines), and medical findings MF.3' modified based on a prior user modification UM are displayed in a third way (here, using dashed-dotted lines). There are various other possibilities to notify a user about adoptions of the results, e.g., by using different colors or by displaying notifications and/or warnings in the user interface or using a pop-up window.

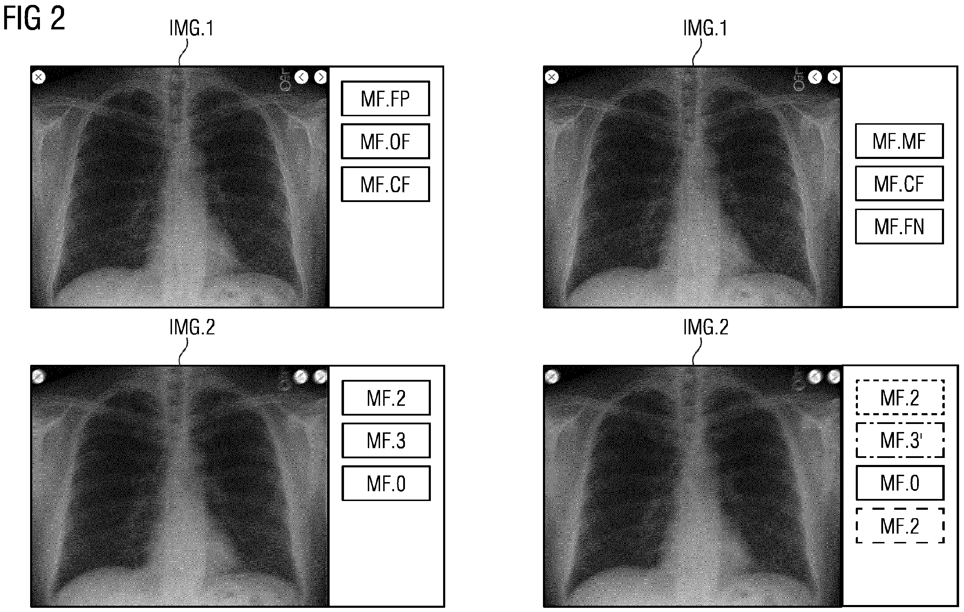

Fig. 2 displays other embodiments of medical images IMG.1, IMG.2 and medical findings MF.FP, MF.FN, MF.OF, MF.MF, MF.CF, MF.0, ..., MF.3.. Here the medical images IMG.1, IMG.2 are two-dimensional X-ray images of a chest of a human, wherein the first medical image IMG.1 was acquired at a first time, and the second medical image IMG.2 was acquired at a second time. The medical findings MF.FP, MF.FN, MF.OF, MF.MF, MF.CF, MF.0, ..., MF.3 are findings that are not associated with a certain location or position within the first medical image IMG.1 or the second medical image IMG.2, but describe a general condition of the patient PAT that can be determined based on the first medical image IMG.1 or the second medical image IMG.2. Otherwise, the first medical image IMG.1 and the second medical image IMG.2 as well as the medical findings MF.FP, MF.FN, MF.OF, MF.MF, MF.CF, MF.0, ..., MF.3 can comprise all advantageous features and embodiments as described with respect to Fig. 1.

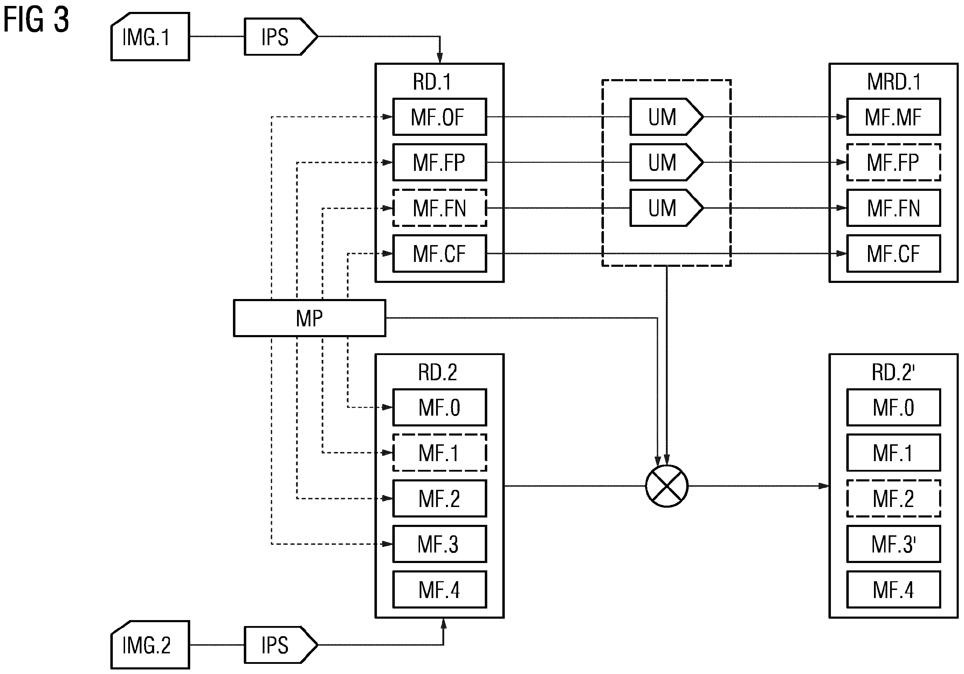

Fig. 3 displays a data flow diagram according to an embodiment of the invention. In this embodiment an image processing system IPS is used at a first time to determine a first result dataset RD.1 based on a first medical image IMG.1, and the image processing system IPS is used at a second time to determine a second result dataset RD.2 based on a second medical image IMG.2. The first medical image IMG.1 and the second medical image IMG.2 are of the same type and relate to the same patient. In this embodiment, both the first medical image IMG.1 and the second medical image IMG.2 are two-dimensional x-ray images of the chest of a patient ("Chest X-Ray"), wherein the second medical image IMG.2 is acquired at a follow-up exam. However, it is also possible to use other types of medical images (e.g., computed tomography, magnetic resonance tomography, ultrasound) or other parts of the human body (e.g., head, abdomen, extremities) in the described embodiment of the invention.

The first result dataset RD.1 comprises several medical findings MF.OF, MF.FP, MF.MF. In this embodiment, the medical findings MF.OF, MF.FP, MF.CF are associated with a certain location or coordinates within the first medical image IMG.1. For example, a medical finding MF.OF, MF.FP, MF.CF could be a candidate lesion or an region of interest corresponding to a candidate lesion. The medical finding MF.OF, MF.FP, MF.CF can comprise further data, e.g. a classification or a severity of the medical finding MF.OF, MF.FP, MF.CF (e.g., a degree of malignancy), a measure related to the medical finding MF.OF, MF.FP, MF.CF (e.g., the area or the volume of the medical finding MF.OF, MF.FP, MF.CF) and/or a segmented area or volume corresponding to the medical finding MF.OF, MF.FP, MF.MF.

After being determined by the image processing system IPS, the first result dataset RD.1 is subject for review by a user (e.g., a radiologist), who reviews the medical findings. Based on user modifications UM submitted by the user, a modified first result dataset MRD.1 can be generated. There are several possibilities of such user modifications UM that can be submitted by the user.

In a first example, there can be an original medical finding MF.OF in the first result dataset RD.1 that is modified by the user to a modified medical finding MF.MF, so that the modified first result dataset MRD.1 does contain the modified medical finding MF.MF and not the original medical finding. In other words, in the process of generating the modified first result dataset MRD.1 the original medical finding MF.OF is removed and the modified medical finding MF.MF is included. In the context of the described embodiment, such a user modification UM can change, e.g., the classification of the detected candidate lesion or the segmented area of the detected candidate lesion.

In a second example, there can be a false positive medical finding MF.FP in the first result dataset RD.1 that is removed by the user. In other words, in the process of generating the modified first result dataset MRD.1 the false positive medical finding MF.FP is removed, and there is no corresponding medical finding in the modified first result dataset MRD.1 (indicated by the dotted lines for the false positive finding MF.FP). In the context of the described embodiment, the user can decide that candidate lesion does not correspond to an actual lesion and remove the candidate lesion from the results by a user modification UM.

In a third example, there can be a false negative medical finding MF.FN that has not been detected by the image processing system IPS in the first image IMG.1, and is not contained in the first result dataset (indicated by the dotted lines for the false negative finding MF.FN), and is subsequently included by the user. In other words, in the process of generating the modified first result dataset MRD.1 the false negative medical finding MF.FN is included by the user. In the context of the described embodiment, a user can manually detect a lesion not detected by the image processing system IPS, and include an additional candidate lesion by a user modification UM.

In a fourth example, there can be a confirmed medical finding MF.CF that has correctly been detected by the image processing system IPS in the first image IMG.1, and is contained in both the first result dataset RD.1 and the modified first result dataset MRD.1. The user can indicate a confirmed medical finding MF.CF by actively confirming the medical finding, or alternatively by not changing the respective medical finding. In the context of the described embodiment, all candidate lesions that are not modified by the user are considered as confirmed candidate lesions.

For the second medical image IMG.2, a second result dataset RD.2 is generated by the image processing system IPS, wherein the second result dataset RD.2 comprises several medical findings MF.0, ..., MF.4. In this embodiment, this second result dataset RD.2 is modified based on a comparison of the first result dataset RD.1 and the modified first result dataset MRD.1 and based on a comparison of the first result dataset RD.1 and the second result dataset RD.2. By comparing the modified first result dataset MRD.1 and the first result dataset RD.1 the user modifications UM can be determined.

In this embodiment, within the comparison of the first result dataset RD.1 and the second result dataset RD.2 a mapping MP is established between the medical findings MF.OF, MF.FP, MF.CF of the first result dataset RD.1 and the medical findings MF.0, ..., MF.4 of the second result dataset RD.2. This mapping MP is not necessarily a one-to-one correspondence, there can be medical findings MF.OF, MF.FP, MF.CF of the first result dataset RD.1 not being mapped to a medical finding MF.0, ..., MF.4 of the second result dataset RD.2, and there can be medical findings MF.0, ..., MF.4 of the second result dataset RD.2 not mapped to a medical finding MF.OF, MF.FP, MF.CF of the first result dataset RD.1. In the context of this embodiment, a registration between the first medical image IMG.1 and the second medical image IMG.2 is established, which can be used for mapping the position of a candidate lesion within the first medical image IMG.1 into a position of the second medical image IMG.2 for creating a mapping of the candidate lesions. Note that for establishing a registration it is not necessary to have access to the first medical image IMG.1, for example by using a landmark-based registration based on landmarks stored in the first result dataset RD.1.

In the first example, if by the respective comparisons it is established that a third medical finding MF.3 in the second result dataset RD.2 is related to the original medical finding MF.OF in the first result dataset RD.1 that has been modified by the user (thereby creating a modified medical finding MF.OM), the third medical finding MF.3 can be adapted automatically according to said modification. In the second example, if by the respective comparisons it is established that a second medical finding MF.2 in the second result dataset RD.2 is related to the false positive medical finding MF.FP in the first result dataset RD.1, the second medical finding MF.2 can be removed. In the third example, if by the respective comparisons it is established that a first medical finding MF.1 related to a false negative medical finding MF.FN in the modified first result dataset MRD.2 is missing in the second result dataset RD.2, it can be included based on the false negative medical finding MF.FN in the modified first result dataset MRD.1. In the fourth example, if by the respective comparisons it is established that a medical finding MF.0, MF.4 in the second result dataset RD.2 is related to a confirmed medical finding MF.CF or cannot be mapped to a medical finding MF.OF, MF.FP, MF.FN, MF.CF of the first result dataset RD.1 or the modified first result dataset MRD.1, it can remain unchanged in the second result dataset RD.2. The result is a modified second result dataset RD.2'.

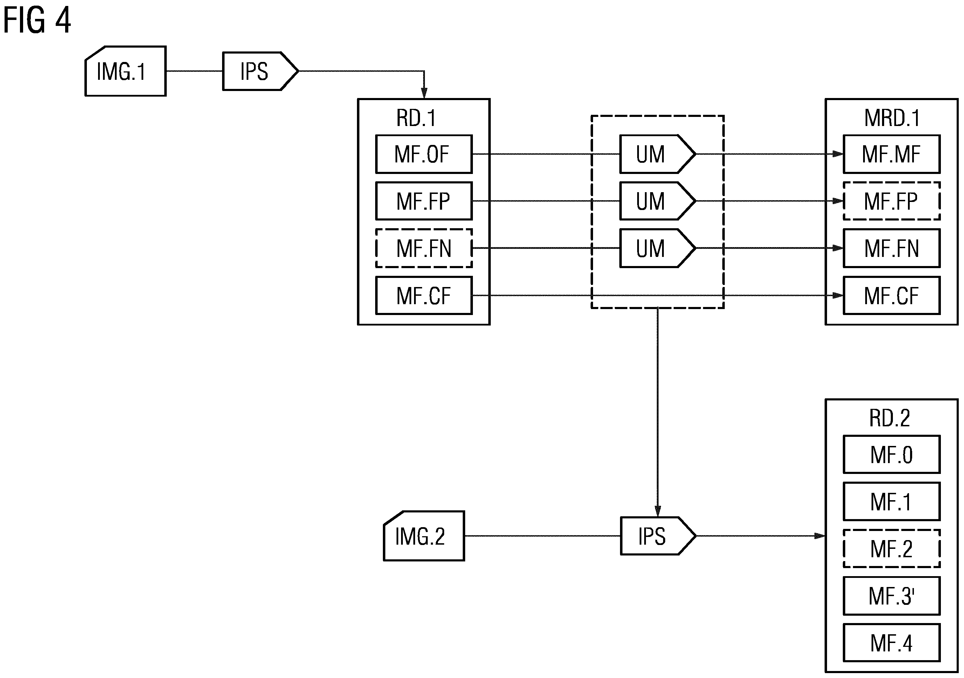

Fig. 4 displays a data flow diagram according to another embodiment of the invention. In this embodiment an image processing system IPS is used at a first time to determine a first result dataset RD.1 based on a first medical image IMG.1, the image processing system IPS is modified and used at a second time to determine a second result dataset RD.2 based on a second medical image IMG.2. The first medical image IMG.1 and the second medical image IMG.2, as well as the first result dataset RD.1 and the modified first result dataset MRD.1 have the same properties and advantageous embodiments as described with respect to Fig. 3.

In this embodiment, the image processing system IPS is modified based on the user modifications UM that were used to transform the first result dataset RD.1 into the modified first result dataset MRD.1, or in other words, based on a comparison of the first result dataset RD.1 and the modified first result dataset MRD.1. In this embodiment, the image processing system IPS comprises a neural network, and modifying the image processing system IPS comprises adopting at least one of the edge weights of the neural network. Details about possible modifications of the image processing system IPS are described later.

In this embodiment, the second result dataset RD.2 is determined by using the second medical image IMG.2 as input for the image processing system IPS after the image processing system IPS has been modified.

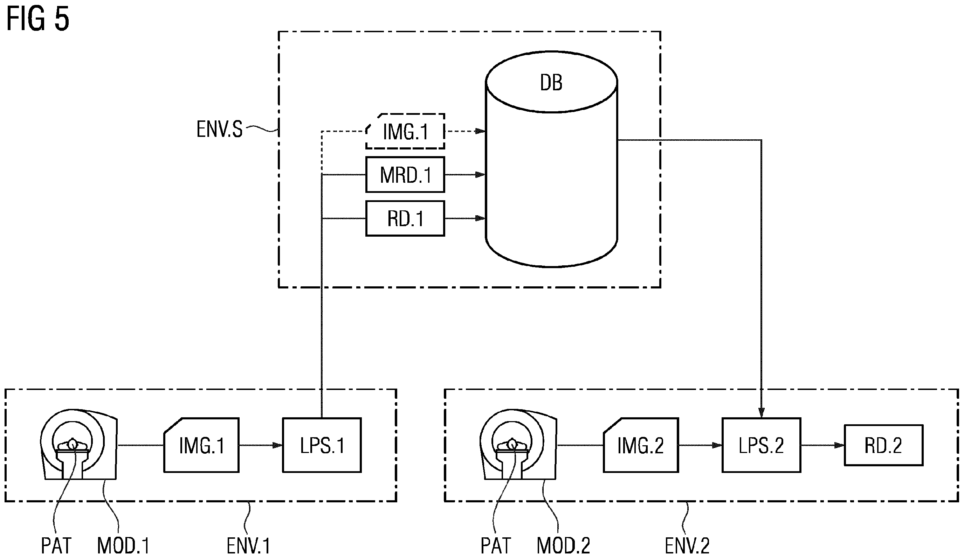

Fig. 5 displays a first embodiment of an infrastructure for methods and systems according to the invention.

In the first embodiment displayed in Fig. 5, there is a first local environment ENV.1 and a second local environment ENV.2. A local environment ENV.1, ENV.2 can be the local IT infrastructure of a medical provided, e.g., a group of hospitals, a single hospital, a department within a hospital or a private practice. In the displayed first embodiment, the first local environment ENV.1 and the second local environment ENV.2 are different environments. Alternatively, the first local environment ENV.1 and the second local environment ENV.2 can be identical.

In the first local environment ENV.1 there is a first medical imaging modality MOD.1, and in the second local environment ENV.2 there is a second medical imaging modality MOD.2. The medical imaging modalities MOD.1, MOD.2 are of the same type. In particular, in the case where the first local environment ENV.1 and the second local environment ENV.2 are identical, also the first medical imaging modality MOD.1 and the second medical imaging modality MOD.2 can be identical. Examples for medical imaging modalities MOD.1, MOD.2 are computed tomography apparatuses, magnetic resonance imaging apparatuses, and X-Ray apparatuses.

In the displayed infrastructure, the first medical imaging modality MOD.1 records the first medical image IMG.1 of the patient PAT within the first local environment ENV.1. The first medical image IMG.1 is locally processed by a first local processing system LPS.1 to generate the first result dataset RD.1, and user modifications UM are received by the first local processing system LPS.1 to generate the modified first result dataset MRD.1.

The first local processing system LPS.1 store the first result dataset RD.1 and the modified first result dataset MRD.1 in an external database DB located in a server environment ENV.S. Optionally, also the first medical image IMG.1 can be stored within the external database DB. In particular, the server environment ENV.S can be a cloud environment or an edge environment. Alternatively, the server environment ENV.S can be identical with the first local environment ENV.1 and/or the second local environment ENV.2.

At a later point in time the second medical imaging modality MOD.2 records the second medical image IMG.2 of the same patient PAT, which is subsequently processed by a second local processing system LPS.2, wherein the second local processing system LPS.2 furthermore accesses the external database DB in order to access the first result dataset RD.1 and the modified first result dataset MRD.1. By processing the second medical image IMG.2 a second result dataset RD.2 is generated, which can also subsequently be stored in the external database DB. In particular, the first local processing system LPS.1 and the second local processing system LPS.2 can be equivalent if the first local environment ENV.1 and the second local environment ENV.2 are equivalent, alternatively, the first local processing system LPS.1 and the second local processing system LPS.2 are separate units.

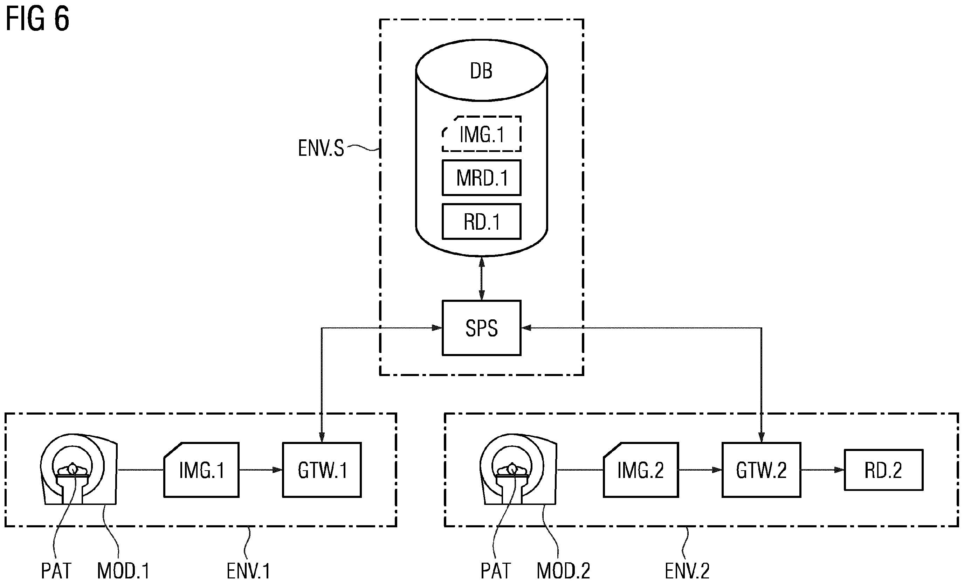

Fig. 6 displays a second embodiment of an infrastructure for methods and systems according to the invention. In contrast to the first embodiment of the infrastructure displayed in Fig. 5, the processing of the medical images IMG.1, IMG.2 is executed within the server environment ENV.S.

In particular, in the local environments ENV.1, ENV.2 there are gateways GTW.1, GTW.2 that forward the medical images IMG.1, IMG.2 to the server environment ENV.S for processing in a server processing system SPS. The gateways GTW.1, GTW.2 can modify the medical images IMG.1, IMG.2 in order to comply with data privacy and data protection requirements, for example, the gateways GTW.1, GTW.2 can anonymize or pseudonymize the medical images IMG.1, IMG.2 before uploading them to the server environment.

In this second embodiment, the server processing system SPS generates the first result dataset RD.1. For determining the modified first result dataset MRD.1, the server processing system SPS can forward the first result dataset RD.1 via the first gateway GTW.1 to the first local environment ENV.1, wherein user modifications UM can be performed to generate the modified first result dataset MRD.1. The modified first result dataset MRD.1 can then be uploaded again via the first gateway GTW.1 to the server environment ENV.S.

As in the first embodiment, the first result dataset RD.1 and the modified first result dataset MRD.1 are stored in an external database DB. In this embodiment, both the server processing unit SPS and the external database DB are located within the same server environment ENV.2. Alternatively, the external database DB could be located in another environment accessible by the server processing unit SPS.

In this second embodiment, the server processing system SPS also generates the second result dataset RD.2 based on the uploaded second medical image IMG.2, the first result dataset RD.1 and the modified first result dataset MRD.2. The second result dataset RD.2 is the forwarded to the second gateway GTW.2 and can be provided within the second local environment ENV.2.

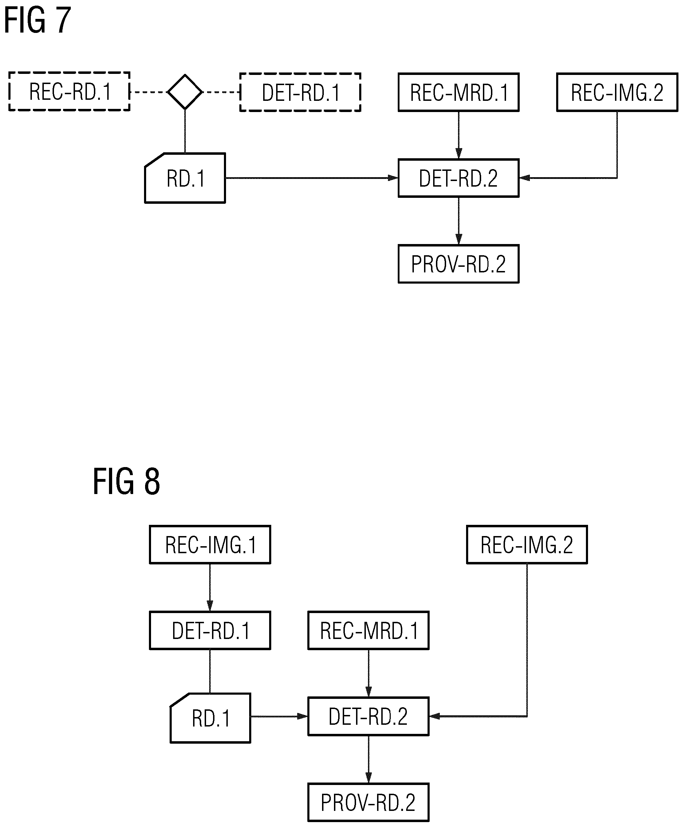

Fig. 7 displays a flowchart of a first embodiment of the method for providing a second result dataset RD.2. In particular, the first embodiment implements the data flow as depicted in and described with respect to Fig. 3 and/or Fig. 4, and can be executed in an infrastructure as described with respect to Fig. 5 and/or Fig. 6. The first medical image IMG.1 and the second medical image IMG.2, as well as the first result dataset RD.1 and the modified first result dataset MRD.1 have the same properties and advantageous embodiments as described with respect to Fig. 3.

The displayed embodiment comprises the step of receiving REC-RD.1 a first result dataset RD.1, or alternatively the step of determining DET-RD.1 the first result dataset RD.1. The first result dataset RD.1 is the output of an image-processing system IPS processing a first medical image IMG.1 of a patient PAT.

In particular, the first result dataset RD.1 can be stored in an external database DB and received REC-RD.1 from this external database DB. In particular, the external database DB can be within a cloud environment, or within a local hospital IT environment. Receiving REC-RD.1 the first result dataset RD.1 can be executed after sending a request for the first result dataset RD.1 to the external database DB. The request can be based on a patient identifier related to the patient PAT, which can be extracted from the second medical image IMG.2.

Another step of the displayed embodiment is receiving REC-MRD.1 a modified first result dataset MRD.1. Here, the modified first result dataset MRD.1 is based on a user modification UM of the first result dataset RD.1.

The modified first result dataset MRD.1 can be stored in the same external database DB as the first result dataset RD.1. In particular, there can be a relation between the modified first result dataset MRD.1 and the first result dataset RD.1, and the relation can also be stored in the external database DB. In particular, the first result dataset RD.1 and the modified first result dataset MRD.1 can share a common identifier that can be based on an identifier of the patient.

Another step of the displayed embodiment is receiving REC-IMG.2 a second medical image IMG.2 of the patient PAT, wherein the first medical image IMG.1 and the second medical image IMG.2 are of the same type.

As displayed in Fig. 7, the order of the steps of receiving REC-RD.1 or determining DET-RD.1 the first result dataset RD.1, receiving the modified first result dataset MRD.1 and receiving REC-IMG.2 the second medical image IMG.2 are independent of each other and can be executed in any order.

In particular, receiving REC-IMG.2 the second medical image IMG.2 can trigger the other steps of the method, and metadata (e.g., contained in certain DICOM tags) can be used for identifying and requesting the first result dataset RD.1 and the modified first result dataset MRD.1.

A further step of the displayed embodiment is determining DET-RD.2 a second result dataset RD.2, RD.2' based on a comparison of the first result dataset RD.1 and the modified first result dataset MRD.1, and based on processing the second medical image IMG.2 with the image-processing system IPS. Details of this step are described with respect to the further embodiments.

The last step of the displayed embodiment is providing PROV-RD.2 the second result dataset RD.2, RD.2'. Providing PROV-RD.2 the second result dataset RD.2, RD.2' can comprise displaying, transmitting and/or storing the second result dataset RD.2, RD.2'. In particular, the second result dataset RD.2, RD.2' can be stored in the same external database DB the first result dataset RD.1 and the modified first result dataset MRD.1 are stored in.

Fig. 8 displays a flowchart of a second embodiment of the method for providing a second result dataset RD.2. In particular, the second embodiment implements the data flow as depicted in and described with respect to Fig. 3 and/or Fig. 4, and can be executed in an infrastructure as described with respect to Fig. 5 and/or Fig. 6. The first medical image IMG.1 and the second medical image IMG.2, as well as the first result dataset RD.1 and the modified first result dataset MRD.1 have the same properties and advantageous embodiments as described with respect to Fig. 3.

In contrast to the first embodiment of the method for providing a second result dataset RD.2, the second embodiment comprises the steps of receiving REC-IMG.1 the first medical image IMG.1 and determining DET-RD.1 the first result dataset RD.1 by processing the first medical image IMG.1 with the image-processing system IPS. In other words, in this second embodiment the first result dataset RD.1 is not received, but directly determined within the method.

The remaining steps of the method, namely receiving REC-MRD.1 the modified first result dataset MRD.1, receiving REC-IMG.2 the second medical image IMG.2 of the patient PAT, determining DET-RD.2 the second result dataset RD.2, RD.2' and providing PROV-RD.2 the second result dataset RD.2, RD.2' are not modified with respect to the first embodiment of the method. In particular, those steps can comprise the same advantageous features as described with respect to Fig. 7.

In the following embodiments of the method for providing a second result dataset RD.2 the first result dataset RD.1 is displayed as an input for the respective flowchart. This is a short notation for the fact that the first result dataset RD.1 has been received or determined as described with respect to the first embodiment as displayed in Fig. 7, or that the first result dataset RD.1 has been determined after receiving the first medical image IMG.1 as described with respect to the second embodiment as displayed in Fig. 8. In other words, those two alternatives for obtaining the first result dataset RD.1 can also be used in the following embodiments.

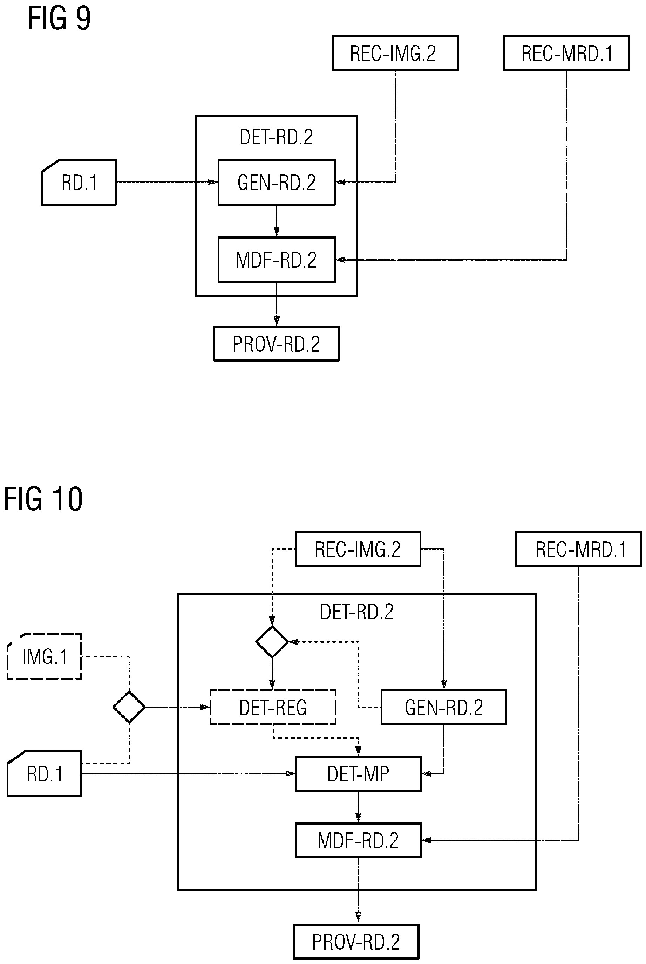

Fig. 9 displays a flowchart of a third embodiment of the method for providing a second result dataset RD.2. In particular, the second embodiment implements the data flow as depicted in and described with respect to Fig. 3, and can be executed in an infrastructure as described with respect to Fig. 5 and/or Fig. 6.

In this third embodiment, the step of determining DET-RD.2 the second result dataset RD.2 comprises generating GEN-RD.2 the second result dataset RD.2 by processing the second medical image IMG.2 with the image-processing system IPS. Furthermore, the step of determining DET-RD.2 the second result dataset RD.2 comprises modifying MDF-RD.2 the second result dataset RD.2 based on the comparison of the first result dataset RD.1 and the modified first result dataset MRD.1. The step of modifying MDF-RD.2 the second result dataset RD.2 is executed after the step of generating GEN-RD.2 the second result dataset RD.2.

In this embodiment, the first result dataset RD.1 comprises a first set of medical findings MF.OF, MF.FP, MF.CF, the modified first result dataset MRD.1 comprises a modified first set of medical findings MF.MF, MF.FN, MF.CF and the second result dataset RD.2 comprises a second set of medical findings MF.0, MF.2, MF.3, MF.4. In the step of generating GEN-RD.2 the second result dataset RD.2 the second medical image IMG.2 is used as an input for the image-processing system IPS, and the image-processing system IPS gives as output the second result dataset RD.2 and/or the second set of medical findings MF.0, MF.2, MF.3, MF.4 when using the second medical image IMG.2 as input.

In this embodiment, the first medical image IMG.1 and the second medical image IMG.2 are two-dimensional X-ray images of the chest of a patient, however, the embodiment of the method can also be used for other types of medical images IMG.1, IMG.2. Furthermore, in this embodiment the medical findings MF.OF, MF.MF, MF.FP, MF.FN, MF.CF, MF.0, MF.2, MF.3, MF.4 correspond to structures within the medical images IMG.1, IMG.2, e.g., candidate lesions within the two-dimensional X-ray images. In particular, the medical findings MF.OF, MF.MF, MF.FP, MF.FN, MF.CF, MF.0, MF.2, MF.3, MF.4 comprise coordinates within the medical image IMG.1, IMG.2 that define a bounding box indicating the location and the size of a candidate lesion. For example, the bounding box can be a rectangular area defined by three points indicating the corners of the rectangle. Alternatively, the medical findings MF.OF, MF.MF, MF.FP, MF.FN, MF.CF, MF.0, MF.2, MF.3, MF.4 can correspond to a finding not associated with a location within the medical images IMG.1, IMG.2, e.g., the presence of pneumonia in the patient. Alternatively, the medical findings MF.OF, MF.FP, MF.CF, MF.0, MF.2, MF.3, MF.4 can correspond to a quantitative measurement related to the medical images IMG.1, IMG.2, e.g., the size of a pneumothorax (i.e. the volume of air in the pleural space) based on a measurement of the distance between the chest wall and the lung.

In particular, in the step of modifying MDF-RD.2 the second result dataset RD.2 based on the comparison of the first result dataset RD.1 and the modified first result dataset MRD.1 the difference between the first set of medical findings MF.OF, MF.FP, MF.CF and the modified first set of medical findings MF.MF, MF.FN, MF.CF is determined. Those differences are based on user modifications UM and are indicative of those user modifications UM. For modifying MDF-RD.2 the second result dataset RD.2, the user modifications UM that resulted in the modified first result dataset MRD.1 are repeated for the second result dataset RD.2.

Fig. 10 displays a flowchart of a fourth embodiment of the method for providing a second result dataset RD.2. In particular, the second embodiment implements the data flow as depicted in and described with respect to Fig. 3, and can be executed in an infrastructure as described with respect to Fig. 5 and/or Fig. 6.

In this fourth embodiment, the step of determining DET-RD.2 the second result dataset RD.2 furthermore comprises determining DET-MP a mapping MP between medical findings MF.OF, MF.FP, MF.CF contained in the first result dataset RD.1 and medical findings MF.0, MF.2, MF.3, MF.4 contained in the second result dataset RD.2.

In this embodiment, a mapping MP assigns one of the medical findings MF.OF, MF.FP, MF.CF contained in the first result dataset RD.1 to one of the medical findings MF.0, MF.2, MF.3, MF.4 contained in the second result dataset RD.2, while it is possible that some of the medical findings MF.OF, MF.FP, MF.CF contained in the first result dataset RD.1 are not mapped at all and/or that some of the medical findings MF.0, MF.2, MF.3, MF.4 contained in the second result dataset RD.2 are not mapped at all. In other word, a mapping MP consists of pairs of medical findings, the first element corresponding to a medical finding MF.OF, MF.FP, MF.CF contained in the first result dataset RD.1 and the second element corresponding to a medical finding MF.0, MF.2, MF.3, MF.4 contained in the second result dataset RD.2, wherein both the first element and the second element can be empty.

Additionally, the mapping MP can also be established for a medical finding MF.FN not contained in the first result dataset RD.1, but included into the modified first result dataset MRD.1 by a user modification UM.

Furthermore, in this embodiment modifying MDF-RD.2 the second result dataset RD.2 is based on the mapping MP previously determined. In particular, user modifications UM related to the first result dataset RD.1 can be translated to the second result dataset RD.2 based on the mapping MP. More particularly, a user modification UM related to one of the medical findings MF.OF, MF.FP, MF.CF contained in the first result dataset RD.1 can be translated to the respective mapped medical finding MF.0, MF.2, MF.3, MF.4 contained in the second result dataset RD.2.

In this fourth embodiment, the step of determining DET-RD.2 the second result dataset RD.2 furthermore comprises determining DET-REG a registration between the first medical image IMG.1 and the second medical image IMG.2 based on the first result dataset RD.1 and the second result dataset RD.2 and/or based on the first medical image IMG.1 and the second medical image IMG.2, and the mapping MP between medical findings contained in the first result dataset RD.1 and medical findings contained in the second result dataset RD.2 is based on said registration.

Alternatively, the mapping MP can also be established without a registration between the first medical image IMG.1 and the second medical image IMG.2. For example, if it can be assumed that the first medical image IMG.1 and the second medical image IMG.2 are already aligned reasonably well, a medical finding in the first medical image IMG.1 can be mapped to the medical finding in the second medical image IMG.2 that is located closest to the pixel-based or voxel-based location of the medical finding in the first medical image IMG.1, if the Euclidean distance between the respective coordinates is below a predefined threshold (wherein coordinates in the first medical image IMG.1 are assumed to match coordinates in the second medical image IMG.2).

In this fourth embodiment, the registration is a non-rigid registration based on a vector momentum-parameterized stationary velocity field. Methods for determining such a registration function are known e.g. from the paper Z. Shen et al. "Networks for Joint Affine and Non-Parametric Image Registration" (20019) 4219-4228, 10.1109/CVPR.2019.00435.

In particular, if v denotes the vector field, $\Phi$ denotes the registration function (also denoted as registration map), $I_1$ denotes the first medical image IMG.1 and $I_2$ denotes the second medical image IMG.2, the registration function $\Phi^{-1}$ can be determined by minimizing

$$m^* = \mathrm{argmin}_{m0}\ \lambda_{vf}\ \langle m_0,\ v_0 \rangle + \mathrm{sim}[I_1 \circ \Phi(1),\ I_2].$$

The initial or boundary conditions are given by:

$$\Phi^{-1}{}_t + D\Phi v = 0;$$

$$\Phi^{-1}(0) = \Phi_{(0)};$$

$$v_0 = (L^+L)^{-1} m_0.$$

Here, D denotes the Jacobian and v0 = <L$^+$Lv, v> is a spatial norm defined by specifying the differential operator L and its adjoint L$^+$. Picking a specific L implies picking an expected model of deformation. In vSVF, the differential operator is spatially invariant and is predefined to encode a desired level of smoothness. The vector-valued momentum m is both spatio-temporal invariant and is equivalent to m = L$^+$Lv. The optimization takes places on m, where the velocity is smoothed from it. The resulting transformation is guaranteed to be diffeomorphic.

Alternatively, other image-based registration methods can be used. Alternatively, a registration can also be performed without having access to the medical images IMG.1, IMG.2 based on the first result dataset RD.1 and the second result dataset RD.2. For example, the first result dataset RD.1 and the second result dataset RD.2 can comprise coordinates for certain specific landmarks in the medical images IMG.1, IMG.2, so that a landmark-based registration method can be used, or a combination of image-based registration methods and landmark-based registration methods (e.g., as proposed in H. J. Johnson and G. E. Christensen, "Consistent landmark and intensity-based image registration," in IEEE Transactions on Medical Imaging, vol. 21 (2002), pp. 450-461, doi: 10.1109/TMI.2002.1009381). Such different types of registration methods are well-known for the person skilled in the art (e.g., see L. Brown, "A survey of image registration techniques", in ACM Computing Surveys, vol. 24 (1992), pp 325-376, doi: 10.1145/146370.146374).

The registration can be used for determining DET-MP the mapping MP in cases where medical findings are associated with a location or coordinates within the medical images IMG.1, IMG.2. For example, let Φ denote the registration function (so that Φ(x) are coordinates in the second medical image IMG.2 that correspond, according to the registration, to coordinates x in the first medical image IMG.1) and $x_i$ denote the coordinates of the i-th medical finding (with $1 \leq i \leq I$) in the first medical image IMG.1. Then Φ($x_i$) are the corresponding coordinates of the i-th medical finding in the second medical image IMG.2. Furthermore, let $y_j$ denote the coordinates of the j-th medical finding (with $1 \leq j \leq J$) in the second medical image IMG.2. The i-the medical finding in the first medical image IMG.1 can then be mapped to the j-th medical finding in the second medical image IMG.2 where (Φ($x_i$) - $y_j$)$^2$ is minimal, if (Φ($x_i$) - $y_j$)$^2$ is smaller than a predefined threshold, and to no medical finding otherwise (in other words, the nearest medical finding with respect to the registration, if it is nearer than a predefined threshold). Alternatively, it is also possible to do the mapping MP not separately for each medical finding, but at the same time for all medical findings, by minimizing the sum of pairwise distances.

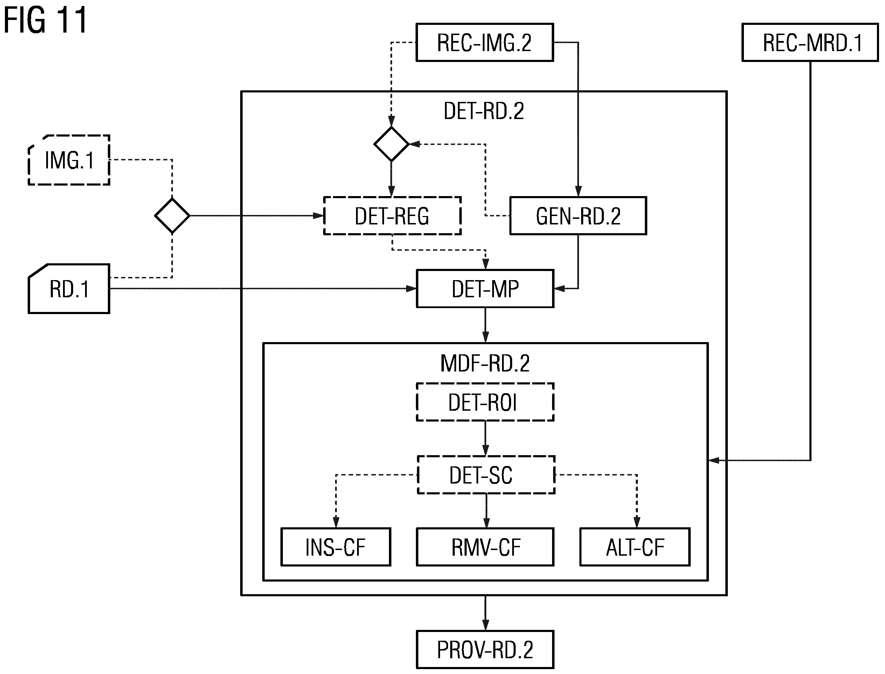

Fig. 11 displays a flowchart of a fifth embodiment of the method for providing a second result dataset RD.2. In particular, the second embodiment implements the data flow as depicted in and described with respect to Fig. 3, and can be executed in an infrastructure as described with respect to Fig. 5 and/or Fig. 6.

In this fifth embodiment, the step of modifying MDF-RD.2 the second result dataset RD.2 comprises at least one of inserting INS-MF a first medical finding MF.1 into the second result dataset RD.2, removing RMV-MF a second medical finding MF.2 from the second result dataset RD.2, and/or altering ALT-MF a third medical finding MF.3 within the second result dataset RD.2.

The first medical finding MF.1 to be inserted into the second result dataset RD.2 corresponds (by means of the mapping MP) to a false negative medical finding MF.FN, or in other words, a medical finding MF.FN not contained in the first result dataset RD.1, but contained in the modified first result dataset MRD.1. In particular, the corresponding position of the false negative medical finding MF.FN in the second medical image IMG.2 can be calculated based on the registration or transformation between the first medical image IMG.1 and the second medical image IMG.2, and the first medical image IMG.1 can comprise the transformed position within the second medical image IMG.2 and all the other information of the false negative medical finding MF.FN. For example, if the false negative medical finding MF.FN corresponds to a lung nodule manually marked by the physician within the user modification UM, the first medical finding MF.1 can indicate that there is lung nodule at the corresponding location within the second medical image IMG.2. If the false negative medical finding MF.FN furthermore comprises a classification of the lung nodule (e.g., a level of benignancy or malignancy), the first medical finding MF.1 can comprise the same classification of the lung nodule.

In particular, if there is a first medical finding MF.1 inserted into the second result dataset RD.2, when providing PROV-RD.2 or displaying the second result dataset RD.2 an indication can be provided or displayed that the first medical finding MF.1 was inserted based on a user modification UM of the first result dataset RD.1. For example, the first medical finding MF.1 can be displayed in a certain color or with a certain symbol.

The second medical finding MF.2 to be removed from the second result dataset RD.2 corresponds (by means of the mapping MP) to a false positive medical finding MF.FP, or in other words, a medical finding MF.FP contained in the first result dataset RD.1, but not contained in the modified first result dataset MRD.1. For example, if the false positive medical finding MF.FP corresponds to a lung nodule falsely detected by the imaging processing system IPS within the first medical image IMG.1 and subsequently removed by the physician within the user modification UM (since the detected structure does not correspond to a lung nodule, but to an unsuspicious other structure), and the second medical finding MF.2 corresponds to a similar unsuspicious structure, the second medical finding MF.2 can be removed from the second result dataset RD.2

In particular, if there is a second medical finding MF.2 removed from the second result dataset RD.2, when providing PROV-RD.2 or displaying the second result dataset RD.2 an indication can be provided or displayed that the second medical finding MF.2 was removed based on a user modification UM of the first result dataset RD.1. For example, the second medical finding MF.2 can be displayed in a certain color or with a certain symbol, for example, in a lighter color than another medical finding not removed.

The third medical finding MF.3 to be altered within the second result dataset RD.2 corresponds (by means of the mapping MP) to an original medical finding MF.OF within the first result dataset RD.1 and a corresponding modified medical finding MF.MF within the modified first result dataset MRD.1. In particular, the third medical finding is altered in accordance with the modification of the original medical finding MF.OF that resulted in the modified medical finding MF.MF. For example, if the original medical finding MF.OF corresponds to a lung nodule classified by the image processing system IPS with a first value (e.g., related to its level of malignancy or benignancy), and the classification was altered by the physician to a second value by means of a user modification UM, the corresponding value of the third medical finding MF.3 can be changed to the second value. In another example, if the original medical finding MF.OF corresponds to a segmentation of a structure within the first medical image IMG.1, and the segmentation was altered by the physician ny means of a user modification UM resulting in the modified medical finding MF.MF, a segmentation within the third medical finding MF.3 can be adopted accordingly. In particular, a registration and/or a transformation between the first medical image IMG.1 and the second medical image IMG.2 can be used to map the segmentation within the modified medical finding MF.MF to the second medical image IMG.2 and replace the respective segmentation within the third medical finding MF.3, thereby altering the third medical finding.

In particular, if there is a third medical finding MF.3 altered within the second result dataset RD.2, when providing PROV-RD.2 or displaying the second result dataset RD.2 an indication can be provided or displayed that the third medical finding MF.3 was altered based on a user modification UM of the first result dataset RD.1. For example, the third medical finding MF.3 can be displayed in a certain color or with a certain symbol, or some original and altered values can be displayed simultaneously.

In this fifth embodiment the step of modifying MDF-RD.2 the second result dataset RD.2 furthermore and optionally comprises determining DET-ROI a first region within the first medical image IMG.1 and a second region within the second medical image IMG.2, wherein the first region and the second region correspond to the first medical finding MF.1 to be inserted, the second medical finding MF.2 to be removed and/or the third medical finding MF.3 to be altered.

In this embodiment, the first region within the first medical image IMG.1 is a quadratic or cubic region with a predefined size (measured in numbers of pixels or voxels) centered at the location of the respective medical finding MF.FN, MF.FP, MF.OF, MF.MF within the first medical image IMG.1. Furthermore, the second region within the second medical image IMG.2 is a quadratic or cubic region with the same predefined size (measured in numbers of pixels or voxels) centered at the location of the respective medical finding MF.1, MF.2, MF.3 within the second medical image IMG.2. Alternatively, other shapes of the first region and the second region can be used.

Furthermore, the step of modifying MDF-RD.2 the second result dataset RD.2 furthermore and optionally comprises determining DET-SC a similarity score between the first region and the second region, wherein the step of inserting INS-MF the first medical finding MF.1, the step of removing RMV-MF the second medical finding MF.2 and/or altering ALT-MF the third medical finding MF.3 is executed only if the similarity score fulfills a predefined criterion.

In this embodiment, the similarity score is based on the pixel-wise or voxel-wise squared difference of the first region and the second region (wherein the difference is the difference of the intensity values of the respective pixels or voxels). In particular, the similarity score can be normalized by the number of pixels or voxels within the first region and the second region. In this embodiment, the predefined criterion is a upper threshold for the similarity score, so that the step of inserting INS-MF the first medical finding MF.1, the step of removing RMV-MF the second medical finding MF.2 and/or altering ALT-MF the third medical finding MF.3 is executed if the similarity score is below the threshold, and not executed of the similarity score is above the threshold.

Alternatively determining DET-SC the similarity score can comprise using the first region and the second region as input data for a similarity-detecting machine learning algorithm, wherein the similarity-detecting machine learning algorithm is based on training data comprising pairs of original regions and transformed regions. In particular, the similarity-detecting machine learning algorithm takes as input two regions of the same size and gives as output a probability value between 0 and 1, wherein 1 corresponds to a high similarity and 0 corresponds to a low similarity. The similarity-detecting machine learning algorithm can be trained based on pairs of regions, wherein the ground-truth is chosen to be 1 if the training input regions correspond to the same region (up to a transformation), and wherein the ground-truth is chosen to be 0 if the training input regions correspond to different regions. In this alternative, the predefined criterion is a lower threshold for the similarity score (being the output value of the similarity-detecting machine learning algorithm), so that the step of inserting INS-MF the first medical finding MF.1, the step of removing RMV-MF the second medical finding MF.2 and/or altering ALT-MF the third medical finding MF.3 is executed if the similarity score is above the threshold, and not executed of the similarity score is below the threshold.

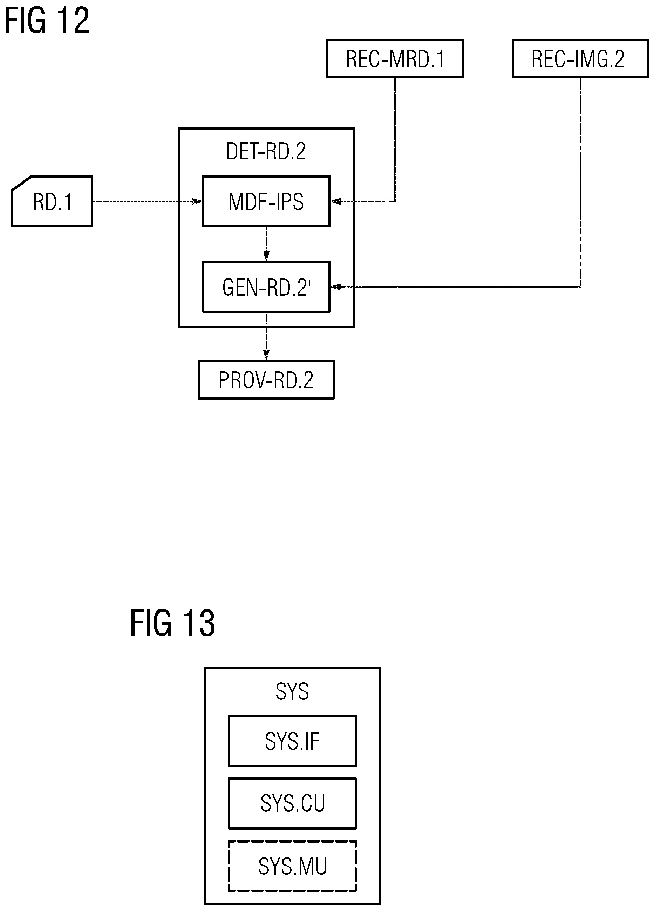

Fig. 12 displays a flowchart of a sixth embodiment of the method for providing a second result dataset RD.2. In particular, the first embodiment implements the data flow as depicted in and described with respect to Fig. 4, and can be executed in an infrastructure as described with respect to Fig. 5 and/or Fig. 6. The first medical image IMG.1 and the second medical image IMG.2, as well as the first result dataset RD.1 and the modified first result dataset MRD.1 have the same properties and advantageous embodiments as described with respect to Fig. 3.

In this sixth embodiment, the step of determining DET-RD.2 the second result dataset RD.2 comprises modifying MDF-IPS the image-processing system IPS based on the comparison of the first result dataset RD.1 and the modified first result dataset MRD.1, and generating GEN-RD.2' the second result dataset RD.2 by processing the second medical image IMG.2 with the image-processing system IPS. In this embodiment, the step of generating GEN-RD.2' the second result dataset RD.2 is executed after the step of modifying MDF-IPS the image-processing system IPS.

In this embodiment, the step of modifying MDF-IPS the image-processing system IPS comprises at least one of overfitting the trainable machine-learning algorithm based on the comparison of the first result dataset RD.1 and the modified first result dataset MRD.1, altering the operating point of the trainable machine-learning algorithm based on the comparison of the first result dataset RD.1 and the modified first result dataset MRD.1; and/or conditioning the output of the trainable machine-learning algorithm based on based on the comparison of the first result dataset RD.1 and the modified first result dataset MRD.1.

In particular, overfitting the trainable machine-learning algorithm can comprise performing at least one training step using the first medical image IMG.1 as input data, and comparing the output of the trainable machine-learning algorithm with the modified first result dataset MRD.1 or with the differences between the modified first result dataset MRD.1 and the first result dataset RD.1. Subsequently, based on the comparison parameters of the trainable machine-learning algorithm can be adapted, e.g., by using the backpropagation algorithm for a neural network. In particular, the first medical image IMG.1 can be used more frequently than other training data before, accepting less predictive power for general cases, but better results for images that are similar like the first medical image IMG.1 (for example, the second medical image IMG.2).

In addition to or as an alternative to overfitting, the operating point of the trainable machine-learning algorithm can be modified based on the comparison of the first result dataset RD.1 and the modified first result dataset MRD.1. In particular, by the comparison of the first result dataset RD.1 and the modified first result dataset MRD.1 a number of false positive medical findings MF.FP and a number of false negative medical findings MF.FN can be determined. If the number of false positive medical findings MF.FP is higher than the number of false negative medical findings MF.FN the sensitivity can decreased and the specificity can be increased by moving the operating point, and vice versa.

Fig. 13 displays a providing system SYS for providing a second result dataset RD.2, RD.2'. The providing system SYS comprises an interface SYS.IF, a computation unit SYS.CU and a memory unit SYS.MU.

The providing system SYS can be a (personal) computer, a workstation, a virtual machine running on host hardware, a microcontroller, or an integrated circuit. In particular, the providing system SYS can be mobile devices, e.g., a smartphone or a tablet. As an alternative, the providing system SYS can be a real or a virtual group of computers (the technical term for a real group of computers is "cluster", the technical term for a virtual group of computers is "cloud").

An interface SYS.IF can be embodied as a hardware interface or as a software interface (e.g. PCIBus, USB or Firewire). In particular, the interface SYS.IF can be a combination of several other interfaces, in particular, the interface SYS.IF can comprise one or more interfaces as subcomponent.

In general, a computation unit SYS.CU can comprise hardware elements and software elements, for example a microprocessor, a CPU (acronym for "central processing unit"), a GPU (acronym for "graphical processing unit"), a field programmable gate array (an acronym is "FPGA") or an ASIC (acronym for "application-specific integrated circuit"). The computation unit SYS.CU can be configured for multithreading, i.e. the computation unit SYS.CU can host different computation processes at the same time, executing the either in parallel or switching between active and passive computation processes. In particular, the computation unit SYS.CU can be a combination of several other computation units, in particular, the computation unit SYS.CU can comprise one or more computation units as subcomponents. A memory unit SYS.MU can be e.g. non-permanent main memory (e.g. random access memory) or permanent mass storage (e.g. hard disk, USB stick, SD card, solid state disk).

The providing system SYS can be configured to execute the method according to the invention and/or according to the embodiments displayed in Fig. 7 to Y6. In particular, the providing system SYS can comprise, be part of or be identical with a first local processing system LPS.1, a second local processing system LPS.2 and/or a server processing system SPS.

**Claims**

**1.** A computer-implemented method for providing a second result dataset (RD.2, RD.2'), comprising:

- receiving (REC-RD.1) and/or determining (DET-RD.1) a first result dataset (RD.1),
wherein the first result dataset (RD.1) is the output of an image-processing system (IPS) processing a first medical image (IMG.1) of a patient (PAT),
- receiving (REC-MRD.1) a modified first result dataset (MRD.1),
wherein the modified first result dataset (MRD.1) is based on a user modification (UM) of the first result dataset (RD.1),
- receiving (REC-IMG.2) a second medical image (IMG.2) of the patient (PAT),
wherein the first medical image (IMG.1) and the second medical image (IMG.2) are of the same type,
- determining (DET-RD.2) a second result dataset (RD.2, RD.2') based on a comparison of the first result dataset (RD.1) and the modified first result dataset (MRD.1), and based on processing the second medical image (IMG.2) with the image-processing system (IPS),
- providing (PROV-RD.2) the second result dataset (RD.2, RD.2') .

2. The method according to claim 1, furthermore comprising:

- receiving (REC-IMG.1) the first medical image (IMG.1),
- determining (DET-RD.1) the first result dataset (RD.1) by processing the first medical image (IMG.1) with the image-processing system (IPS).

3. The method according to claim 1 or claim 2, wherein the step of determining (DET-RD.2) the second result dataset (RD.2, RD.2') comprises:

- generating (GEN-RD.2) the second result dataset (RD.2) by processing the second medical image (IMG.2) with the image-processing system (IPS),
- modifying (MDF-RD.2) the second result dataset (RD.2) based on the comparison of the first result dataset (RD.1) and the modified first result dataset (MRD.1),
wherein the step of modifying (MDF-RD.2) the second result dataset (RD.2) is executed after the step of generating (GEN-RD.2) the second result dataset (RD.2).

4. The method according to claim 3, wherein the step of determining (DET-RD.2) the second result dataset (RD.2, RD.2') furthermore comprises:

- determining (DET-MP) a mapping (MP) between medical findings (MF.FP, MF.FN, MF.OF, MF.MF, MF.CF) contained in the first result dataset (RD.1) and/or in the modified first result dataset (MRD.1) and medical findings (MF.0, MF.2, ..., MF.3) contained in the second result dataset (RD.2),
wherein the step of modifying (MDF-RD.2) the second result dataset (RD.2) is based on said mapping (MP),
in particular, wherein the medical findings (MF.FP, MF.OF, MF.CF, MF.0, MF.2, ..., MF.3) are candidate structures, in particular, wherein the candidate structures are indicative of candidate lesions within the patient.

5. The method according to claim 4, wherein the step of determining (DET-RD.2) the second result dataset (RD.2, RD.2') furthermore comprises:

- determining (DET-REG) a registration between the first medical image (IMG.1) and the second medical image (IMG.2) based on the first result dataset (RD.1) and the second result dataset (RD.2), and/or based on the first medical image (IMG.1) and the second medical image (IMG.2),
wherein the mapping (MP) between medical findings (MF.FP, MF.OF, MF.CF) contained in the first result dataset (RD.1) and medical findings (MF.0, MF.2, ..., MF.3) contained in the second result dataset (RD.2) is based on said registration.

6. The method according to claim 5, wherein the registration and/or the mapping (MP) is based on a deformable transformation, in particular, wherein the registration and/or the mapping (MP) is based on a vector momentum-parameterized stationary velocity field.

7. The method according to one of the claims 3 to 6, wherein the step of modifying (MDF-RD.2) the second result dataset (RD.2, RD.2') comprises at least one of:

- inserting (INS-MF) a first medical finding (MF.1) into the second result dataset (RD.2),
- removing (RMV-MF) a second medical finding (MF.2) from the second result dataset (RD.2), and/or

- altering (ALT-MF) a third medical finding (MF.3) within the second result dataset (RD.2).

8. The method according to claim 7, wherein the step of modifying (MDF-RD.2)the second result dataset (RD.2, RD.2') furthermore comprises:

- determining (DET-ROI) a first region within the first medical image (IMG.1) and a second region within the second medical image (IMG.2), wherein the first region and the second region correspond to the first medical finding (MF.1) to be inserted, the second medical finding (MF.2) to be removed and/or the third medical finding (MF.3) to be altered,
- determining (DET-SC) a similarity score between the first region and the second region, wherein the step of inserting (INS-MF) the first medical finding (MF.1), the step of removing (RMV-MF) the second medical finding (MF.2) and/or altering (ALT-MF) the third medical finding (MF.3) is executed only if the similarity score fulfills a predefined criterion.

9. The method according to claim 8, wherein the step of determining (DET-SC) the similarity score comprises using the first region and the second region as input data for a similarity-detecting machine learning algorithm, in particular, wherein the similarity-detecting machine learning algorithm is based on training data comprising pairs of original regions and transformed regions, the transformed regions being based on applying an image transformation to the original region.

10. The method according to one of the claims 7 to 9,

- wherein the modified first result dataset (MRD.1) comprises a false-negative medical finding (MF.FN) not contained in the first result dataset (RD.1) and wherein the first medical finding (MF.1) corresponds to the false-negative medical finding (MF.FN); and/or
- wherein the first result dataset (RD.1) comprises a false-positive medical finding (MF.FP) not contained in the modified first result dataset (MRD.1) and wherein the second medical finding (MF.2) corresponds to the false-positive medical finding (MF.FP); and/or
- wherein the modified first result dataset (MRD.1) comprises a modified medical finding (MF.MF) corresponding to a modification of an original medical finding (MF.OF) contained in the first result dataset (RD.1), wherein the third medical finding (MF.3) corresponds to the original medical finding (MF.OF) and wherein in the step of altering (ALT-MF) the third medical finding (MF.3) is performed in accordance with the modification of the original medical finding (MF.OF).

11. The method according to claim 1 or claim 2, wherein the step of determining (DET-RD.2) the second result dataset (RD.2, RD.2') comprises:

- modifying (MDF-IPS) the image-processing system (IPS) based on the comparison of the first result dataset (RD.1) and the modified first result dataset (MRD.1),
- generating (GEN-RD.2') the second result dataset (RD.2) by processing the second medical image (IMG.2) with the image-processing system (IPS), wherein the step of generating (GEN-RD.2') the second result dataset (RD.2) is executed after the step of modifying (MDF-IPS) the image-processing system (IPS).

12. The method according to claim 11, wherein the image-processing system (IPS)comprises a trainable machine-learning algorithm, wherein the step of modifying (MDF-IPS) the image-processing system (IPS) comprises at least one of:

- overfitting the trainable machine-learning algorithm based on the comparison of the first result dataset (RD.1) and the modified first result dataset (MRD.1);
- altering the operating point of the trainable machine-learning algorithm based on the comparison of the first result dataset (RD.1) and the modified first result dataset (MRD.1); and/or
- conditioning the output of the trainable machine-learning algorithm based on based on the comparison of the first result dataset (RD.1) and the modified first result dataset (MRD.1).

13. The method according to one of the preceding claims, wherein the step of providing (PROV-RD.2) the second result dataset (RD.2) comprises providing an indication about modifications of the second result dataset (RD.2) and/or modifications of the image-processing system (IPS).

14. A providing system (SYS) for providing a second result dataset (RD.2, RD.2') comprising an interface (SYS.IF) and a computation unit (SYS.CU),

   - wherein the interface (SYS.IF) and/or the computation unit (SYS.CU) are configured for receiving (REC-RD.1) and/or determining (DET-RD.1) a first result dataset (RD.1), wherein the first result dataset (RD.1) is the output of an image-processing system (IPS) processing a first medical image (IMG.1) of a patient (PAT);
   - wherein the interface (SYS.IF) is configured for receiving (REC-MRD.1) a modified first result dataset (MRD.1), wherein the modified first result dataset (MRD.1)is based on a user modification (UM) of the first result dataset (RD.1);
   - wherein the interface (SYS.IF) is configured for receiving (REC-IMG.2) a second medical image (IMG.2) of the patient (PAT), wherein the first medical image (IMG.1) and the second medical image (IMG.2) are of the same type;
   - wherein the computation unit (SYS.CU) is configured for determining (DET-RD.2) a second result dataset (RD.2, RD.2') based on a comparison of the first result dataset (RD.1) and the modified first result dataset (MRD.1), and based on processing the second medical image (IMG.2) with the image-processing system (IPS),
   - wherein the interface (SYS.IF) is configured for providing (PROV-RD.2) the second result dataset (RD.2, RD.2').

15. Computer program product or a computer-readable storage medium comprising instructions which, when the program is executed by a computer, cause the computer to carry out the method of one of the claims 1 to 13.

**Amended claims in accordance with Rule 137(2) EPC.**

1. A computer-implemented method for providing a second result dataset (RD.2, RD.2'), comprising:

   - receiving (REC-RD.1) and/or determining (DET-RD.1) a first result dataset (RD.1), wherein the first result dataset (RD.1) is the output of an image-processing system (IPS) processing a first medical image (IMG.1) of a patient (PAT),
   - receiving (REC-MRD.1) a modified first result dataset (MRD.1), wherein the modified first result dataset (MRD.1) is based on a user modification (UM) of the first result dataset (RD.1),
   - receiving (REC-IMG.2) a second medical image (IMG.2) of the patient (PAT),

      wherein the first medical image (IMG.1) and the second medical image (IMG.2) are of the same type,
      wherein the second medical image (IMG.2) was acquired at a later time than the first medical image (IMG.1),

   - determining (DET-RD.2) a second result dataset (RD.2, RD.2') based on a comparison of the first result dataset (RD.1) and the modified first result dataset (MRD.1), and based on processing the second medical image (IMG.2) with the image-processing system (IPS),
   - providing (PROV-RD.2) the second result dataset (RD.2, RD.2'), wherein the step of determining (DET-RD.2) the second result dataset (RD.2, RD.2') comprises:
   - generating (GEN-RD.2) the second result dataset (RD.2) by processing the second medical image (IMG.2) with the image-processing system (IPS),
   - modifying (MDF-RD.2) the second result dataset (RD.2) based on the comparison of the first result dataset (RD.1) and the modified first result dataset (MRD.1), wherein the step of modifying (MDF-RD.2) the second result dataset (RD.2) is executed after the step of generating (GEN-RD.2) the second result dataset (RD.2).

2. The method according to claim 1, furthermore comprising:

   - receiving (REC-IMG.1) the first medical image (IMG.1),
   - determining (DET-RD.1) the first result dataset (RD.1) by processing the first medical image (IMG.1) with the image-processing system (IPS).

3. The method according to claim 1 or claim 2, wherein the step of determining (DET-RD.2) the second result dataset (RD.2, RD.2') furthermore comprises:

   - determining (DET-MP) a mapping (MP) between medical findings (MF.FP, MF.FN, MF.OF, MF.MF, MF.CF) contained in the first result dataset (RD.1) and/or in the modified first result dataset (MRD.1) and medical findings (MF.0, MF.2, ..., MF.3) contained in the second result dataset (RD.2),

wherein the step of modifying (MDF-RD.2) the second result dataset (RD.2) is based on said mapping (MP).

4. The method according to claim 3, wherein the medical findings (MF.FP, MF.OF, MF.CF, MF.0, MF.2, ..., MF.3) are candidate structures, in particular, wherein the candidate structures are indicative of candidate lesions within the patient.

5. The method according to claim 3 or claim 4, wherein the step of determining (DET-RD.2) the second result dataset (RD.2, RD.2') furthermore comprises:

- determining (DET-REG) a registration between the first medical image (IMG.1) and the second medical image (IMG.2) based on the first result dataset (RD.1) and the second result dataset (RD.2), and/or based on the first medical image (IMG.1) and the second medical image (IMG.2),

wherein the mapping (MP) between medical findings (MF.FP, MF.OF, MF.CF) contained in the first result dataset (RD.1) and medical findings (MF.0, MF.2, ..., MF.3) contained in the second result dataset (RD.2) is based on said registration.

6. The method according to claim 5, wherein the registration and/or the mapping (MP) is based on a deformable transformation, in particular, wherein the registration and/or the mapping (MP) is based on a vector momentum-parameterized stationary velocity field.

7. The method according to one of the preceding claims, wherein the step of modifying (MDF-RD.2) the second result dataset (RD.2, RD.2') comprises at least one of:

- inserting (INS-MF) a first medical finding (MF.1) into the second result dataset (RD.2),
- removing (RMV-MF) a second medical finding (MF.2) from the second result dataset (RD.2), and/or
- altering (ALT-MF) a third medical finding (MF.3) within the second result dataset (RD.2).

8. The method according to claim 7, wherein the step of modifying (MDF-RD.2)the second result dataset (RD.2, RD.2') furthermore comprises:

- determining (DET-ROI) a first region within the first medical image (IMG.1) and a second region within the second medical image (IMG.2), wherein the first region and the second region correspond to the first medical finding (MF.1) to be inserted, the second medical finding (MF.2) to be removed and/or the third medical finding (MF.3) to be altered,
- determining (DET-SC) a similarity score between the first region and the second region,

wherein the step of inserting (INS-MF) the first medical finding (MF.1), the step of removing (RMV-MF) the second medical finding (MF.2) and/or altering (ALT-MF) the third medical finding (MF.3) is executed only if the similarity score fulfills a predefined criterion.

9. The method according to claim 8, wherein the step of determining (DET-SC) the similarity score comprises using the first region and the second region as input data for a similarity-detecting machine learning algorithm,
in particular, wherein the similarity-detecting machine learning algorithm is based on training data comprising pairs of original regions and transformed regions, the transformed regions being based on applying an image transformation to the original region.

10. The method according to one of the claims 7 to 9,

- wherein the modified first result dataset (MRD.1) comprises a false-negative medical finding (MF.FN) not contained in the first result dataset (RD.1) and wherein the first medical finding (MF.1) corresponds to the false-negative medical finding (MF.FN); and/or
- wherein the first result dataset (RD.1) comprises a false-positive medical finding (MF.FP) not contained in the modified first result dataset (MRD.1) and wherein the second medical finding (MF.2) corresponds to the false-positive medical finding (MF.FP); and/or

- wherein the modified first result dataset (MRD.1) comprises a modified medical finding (MF.MF) corresponding to a modification of an original medical finding (MF.OF) contained in the first result dataset (RD.1), wherein the third medical finding (MF.3) corresponds to the original medical finding (MF.OF) and wherein in the step of altering (ALT-MF) the third medical finding (MF.3) is performed in accordance with the modification of the original medical finding (MF.OF).

11. The method according to one of the preceding claims, wherein the step of providing (PROV-RD.2) the second result dataset (RD.2) comprises providing an indication about modifications of the second result dataset (RD.2) and/or modifications of the image-processing system (IPS).

12. A providing system (SYS) for providing a second result dataset (RD.2, RD.2') comprising an interface (SYS.IF) and a computation unit (SYS.CU),

- wherein the interface (SYS.IF) and/or the computation unit (SYS.CU) are configured for receiving (REC-RD.1) and/or determining (DET-RD.1) a first result dataset (RD.1), wherein the first result dataset (RD.1) is the output of an image-processing system (IPS) processing a first medical image (IMG.1) of a patient (PAT);
- wherein the interface (SYS.IF) is configured for receiving (REC-MRD.1) a modified first result dataset (MRD.1), wherein the modified first result dataset (MRD.1) is based on a user modification (UM) of the first result dataset (RD.1);
- wherein the interface (SYS.IF) is configured for receiving (REC-IMG.2) a second medical image (IMG.2) of the patient (PAT), wherein the first medical image (IMG.1) and the second medical image (IMG.2) are of the same type, wherein the second medical image (IMG.2) was acquired at a later time than the first medical image (IMG.1);
- wherein the computation unit (SYS.CU) is configured for determining (DET-RD.2) a second result dataset (RD.2, RD.2') based on a comparison of the first result dataset (RD.1) and the modified first result dataset (MRD.1), and based on processing the second medical image (IMG.2) with the image-processing system (IPS),
- wherein the interface (SYS.IF) is configured for providing (PROV-RD.2) the second result dataset (RD.2, RD.2');
wherein the step of determining (DET-RD.2) the second result dataset (RD.2, RD.2') comprises:
- generating (GEN-RD.2) the second result dataset (RD.2) by processing the second medical image (IMG.2) with the image-processing system (IPS),
- modifying (MDF-RD.2) the second result dataset (RD.2) based on the comparison of the first result dataset (RD.1) and the modified first result dataset (MRD.1),
wherein the step of modifying (MDF-RD.2) the second result dataset (RD.2) is executed after the step of generating (GEN-RD.2) the second result dataset (RD.2).

13. Computer program product or a computer-readable storage medium comprising instructions which, when the program is executed by a computer, cause the computer to carry out the method of one of the claims 1 to 11.

FIG 1

FIG 2

IMG.1

MF.FP

MF.OF

MF.CF

IMG.2

MF.2

MF.3

MF.0

IMG.1

MF.MF

MF.CF

MF.FN

IMG.2

MF.2

MF.3'

MF.0

MF.2

EP 4 109 463 A1

FIG 3

IMG.1 — IPS

RD.1
- MF.OF
- MF.FP
- MF.FN
- MF.CF

UM
UM
UM

MRD.1
- MF.MF
- MF.FP
- MF.FN
- MF.CF

MP

RD.2
- MF.0
- MF.1
- MF.2
- MF.3
- MF.4

RD.2'
- MF.0
- MF.1
- MF.2
- MF.3'
- MF.4

IMG.2 — IPS

EP 4 109 463 A1

# FIG 4

FIG 5

EP 4 109 463 A1

FIG 6

## FIG 7

```
┌ ─ ─ ─ ─ ─ ─ ┐        ◇        ┌ ─ ─ ─ ─ ─ ─ ┐     ┌──────────────┐   ┌──────────────┐
│  REC-RD.1   │· · · · · · · · ·│  DET-RD.1   │     │  REC-MRD.1   │   │  REC-IMG.2   │
└ ─ ─ ─ ─ ─ ─ ┘                 └ ─ ─ ─ ─ ─ ─ ┘     └──────────────┘   └──────────────┘

              ⬡ RD.1 ⬡ ─────────────────────────────▶ DET-RD.2 ◀───────────

                                                      PROV-RD.2
```

## FIG 8

```
      ┌──────────────┐                                              ┌──────────────┐
      │  REC-IMG.1   │                                              │  REC-IMG.2   │
      └──────────────┘                                              └──────────────┘

      ┌──────────────┐        ┌──────────────┐
      │  DET-RD.1    │        │  REC-MRD.1   │
      └──────────────┘        └──────────────┘

           ⬡ RD.1 ⬡ ──────────▶ DET-RD.2 ◀────────────────────────

                                PROV-RD.2
```

## FIG 9

## FIG 10

# FIG 11

EP 4 109 463 A1

## FIG 12

```
                          ┌──────────────┐          ┌──────────────┐
                          │  REC-MRD.1   │          │  REC-IMG.2   │
                          └──────┬───────┘          └──────┬───────┘
                                 │                         │
        ┌────────────────────────┼─────────────────┐      │
        │      DET-RD.2          │                 │      │
        │      ┌──────────────┐  │                 │      │
 ┌──────┐│      │   MDF-IPS    │◄─┘                 │      │
 │ RD.1 │┼─────►│              │                    │      │
 └──────┘│      └──────┬───────┘                    │      │
        │             │                             │      │
        │             ▼                             │      │
        │      ┌──────────────┐                     │      │
        │      │  GEN-RD.2'   │◄────────────────────┴──────┘
        │      └──────┬───────┘
        └─────────────┼──────────────────────────────┘
                      ▼
               ┌──────────────┐
               │  PROV-RD.2   │
               └──────────────┘
```

## FIG 13

```
        ┌─────────────────────────┐
        │          SYS            │
        │   ┌─────────────────┐   │
        │   │     SYS.IF      │   │
        │   └─────────────────┘   │
        │   ┌─────────────────┐   │
        │   │     SYS.CU      │   │
        │   └─────────────────┘   │
        │   ┌ ─ ─ ─ ─ ─ ─ ─ ─ ┐   │
        │        SYS.MU           │
        │   └ ─ ─ ─ ─ ─ ─ ─ ─ ┘   │
        └─────────────────────────┘
```

Europäisches
Patentamt

European
Patent Office

Office européen
des brevets

# EUROPEAN SEARCH REPORT

Application Number

EP 21 18 1585

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 111 445 440 A (SHANGHAI UNITED IMAGING INTELLIGENCE HEALTHCARE CO LTD) 24 July 2020 (2020-07-24) * The whole document, in particular: Claim 1, Paragraph [0070], Figure 8. * ----- | 1-15 | INV. G16H30/40 G16H50/20 G16H50/70 |
| X | US 2018/101645 A1 (SORENSON JEFFREY L [US] ET AL) 12 April 2018 (2018-04-12) * The whole document, in particular: Paragraphs [0020], [0031], [0032], [0039], [0047], [0058], [0063],[0091], [0119], [0151] – [0163]. * ----- | 1-15 | |
| X | ZHU ZHEYU ET AL: "EasierPath: An Open-Source Tool for Human-in-the-Loop Deep Learning of Renal Pathology", 2 October 2020 (2020-10-02), COMPUTER VISION – ECCV 2020 : 16TH EUROPEAN CONFERENCE, GLASGOW, UK, AUGUST 23-28, 2020 : PROCEEDINGS; PART OF THE LECTURE NOTES IN COMPUTER SCIENCE ; ISSN 0302-9743; [LECTURE NOTES IN COMPUTER SCIENCE; LECT.NOTES COMPUTER], SPRINGER INTERNATIONAL PU, XP047564518, ISBN: 978-3-030-58594-5 [retrieved on 2020-10-02] * The whole document, in particular: Abstract; Section 2; Figure 2. * ----- | 1-15 | |
| X | US 2019/385018 A1 (NGO DINH NHAN [IT] ET AL) 19 December 2019 (2019-12-19) * The whole document, in particular: Paragraphs [0008] – [0012], [0036], [0046] – [0049], [0092];  Figure 3. * ----- | 1-15 | TECHNICAL FIELDS SEARCHED (IPC) G16H G06N |

The present search report has been drawn up for all claims

| Place of search | Date of completion of the search | Examiner |
|---|---|---|
| Munich | 7 December 2021 | Nagele, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 4 109 463 A1

## ANNEX TO THE EUROPEAN SEARCH REPORT
## ON EUROPEAN PATENT APPLICATION NO.

EP 21 18 1585

07-12-2021

| Patent document cited in search report | | Publication date | Patent family member(s) | | Publication date |
|---|---|---|---|---|---|
| CN 111445440 | A | 24-07-2020 | NONE | | |
| US 2018101645 | A1 | 12-04-2018 | AU | 2017342283 A1 | 11-04-2019 |
| | | | CA | 3038848 A1 | 19-04-2018 |
| | | | EP | 3526730 A1 | 21-08-2019 |
| | | | JP | 2019536132 A | 12-12-2019 |
| | | | KR | 20190071724 A | 24-06-2019 |
| | | | US | 2018101645 A1 | 12-04-2018 |
| | | | US | 2019392942 A1 | 26-12-2019 |
| | | | WO | 2018071575 A1 | 19-04-2018 |
| US 2019385018 | A1 | 19-12-2019 | AR | 115522 A1 | 27-01-2021 |
| | | | AU | 2019286544 A1 | 14-01-2021 |
| | | | BR | 112020025390 A2 | 09-03-2021 |
| | | | CA | 3103316 A1 | 19-12-2019 |
| | | | CN | 112543941 A | 23-03-2021 |
| | | | EP | 3582143 A1 | 18-12-2019 |
| | | | EP | 3762868 A1 | 13-01-2021 |
| | | | JP | 2021527549 A | 14-10-2021 |
| | | | KR | 20210032951 A | 25-03-2021 |
| | | | SG | 11202012374W A | 28-01-2021 |
| | | | TW | 202018586 A | 16-05-2020 |
| | | | US | 2019385018 A1 | 19-12-2019 |
| | | | US | 2020226423 A1 | 16-07-2020 |
| | | | US | 2021133507 A1 | 06-05-2021 |
| | | | WO | 2019238712 A1 | 19-12-2019 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Z. SHEN et al.** *Networks for Joint Affine and Non-Parametric Image Registration,* 4219-4228
- **H. J. JOHNSON ; G. E. CHRISTENSEN.** Consistent landmark and intensity-based image registration. *IEEE Transactions on Medical Imaging,* 2002, vol. 21, 450-461
- **L. BROWN.** A survey of image registration techniques. *ACM Computing Surveys,* 1992, vol. 24, 325-376